# EUROPEAN PATENT APPLICATION

(11) **EP 4 751 690 A1**
(43) Date of publication of application: **03.06.2026**
(21) Application number: 25218471.8
(22) Date of filing: 25.11.2025
(51) Int. Cl.: A61F 7/02, A61F 7/00

(54) **DRAINAGE METHOD AND DRAINAGE STRUCTURE FOR COLD-HOT COMPRESS DEVICE, AND THE COLD-HOT COMPRESS DEVICE**

(30) Priority: 27.11.2024 CN 202422904505 U; 26.12.2024 CN 202423234976 U; 09.01.2025 US 202519015615; 11.10.2025 CN 202511449614
(71) Applicant: Xiamen Ubest Electronic Technology Co., Ltd., Xiamen, Fujian 361000 (CN)
(72) Inventor: ZHU, Zonghu, Xiamen, 361000 (CN)
(74) Representative: Verscht, Thomas Kurt Albert

(57) **Abstract**

The present disclosure provides a drainage method for a cold-hot compress device, the cold-hot compress device comprises a carrier and a main unit; the carrier comprises an inflatable layer and a working layer, a pumping device is disposed in the main unit, and the pumping device is connected to the inflatable layer and the working layer via a delivery pipe to deliver air to the inflatable layer and deliver liquid or the air to the working layer; and the drainage method comprises a step of delivering the air to the working layer via the pumping device to discharge the liquid in the working layer. The present disclosure further provides a drainage structure adopting the aforementioned method. The present disclosure further provides a cold-hot compress device, it comprises the drainage structure, it further comprises a storage tank and a heat exchanger, the pumping device is used to deliver the liquid in the storage tank to the working layer via the waterway delivery pipe, and the waterway delivery pipe exchanges heat with the heat exchanger.

## Description

### RELATED APPLICATIONS

This application claims priority to US patent application 19/015,615, filed on January 9, 2025, Chinese patent application 202423234976.3, filed on December 26, 2024, Chinese patent application 202511449614.2, filed on October 11, 2025, and Chinese patent application 202422904505.2, filed on November 27, 2024. US patent application 19/015,615, Chinese patent application 202423234976.3, Chinese patent application 202511449614.2, and Chinese patent application 202422904505.2 are incorporated herein by reference.

### FIELD OF THE DISCLOSURE

The present disclosure the field of nursing equipment, and in particular relates to a cold-hot compress device.

### BACKGROUND OF THE DISCLOSURE

Cold-hot compress devices usually transfer temperature to a carrier through components capable of cooling and heating, so that cold-hot compress can be applied to a nursing part attached to the carrier to achieve an effect of treatment and nursing. In the design of the existing cold-hot compress devices, semiconductors are commonly used for cooling and heating, but the semiconductors are mostly applied to small-sized cold-hot compress devices and cannot meet requirements of rapid cooling and heating when large-area cold-hot compress is required; and even if multiple groups of semiconductors are combined to form a large-area working surface, structures of the semiconductors have limitations, cannot be bent arbitrarily, and cannot meet a requirement of being close fitting with the nursing part. At the same time, after the cold-hot compress is complete, liquid in the carrier needs to be drained before the carrier can be folded and stored, however, in the existing techniques, there is a problem that a drainage time is long.

### BRIEF SUMMARY OF THE DISCLOSURE

A main technical problem to be solved by the present disclosure is to provide a drainage method and a structure for a cold-hot compress device, so as to improve a drainage speed.

A secondary technical problem to be solved by the present disclosure is to increase a wrapping area of a carrier of the cold-hot compress device and improve an efficiency of cooling and heating.

In order to solve the aforementioned technical problems, the present disclosure provides a drainage method for a cold-hot compress device, the cold-hot compress device comprises a carrier and a main unit; the carrier comprises an inflatable layer and a working layer, a pumping device is disposed in the main unit, and the pumping device is connected to the inflatable layer and the working layer via a delivery pipe to deliver air to the inflatable layer and deliver liquid or the air to the working layer;

The drainage method comprises a step of delivering the air to the working layer via the pumping device to discharge the liquid in the working layer.

The present disclosure further provides a drainage structure for a cold-hot compress device, the cold-hot compress device comprises a carrier and a main unit, the carrier comprises an inflatable layer and a working layer, a pumping device is disposed in the main unit, the pumping device is respectively connected to the inflatable layer and the working layer via an air path delivery pipe and a waterway delivery pipe;

The pumping device is further connected to the waterway delivery pipe via a control valve, so as to deliver air to the working layer via the waterway delivery pipe when the control valve is opened.

In a preferred embodiment, the control valve is a three-way valve with one inlet and two outlets, the inlet of the three-way valve is in communication with an air outlet of the pumping device, a first outlet is connected to the air path delivery pipe, and a second outlet is connected to the waterway delivery pipe.

In a preferred embodiment, the pumping device comprises an air pump and a water pump, and a water outlet of the water pump is connected to the waterway delivery pipe; or the pumping device is an air-liquid dual-purpose pump.

The present disclosure further provides a cold-hot compress device, it comprises the drainage structure, it further comprises a storage tank and a heat exchanger, the pumping device is used to deliver the liquid in the storage tank to the working layer via the waterway delivery pipe, and the waterway delivery pipe exchanges heat with the heat exchanger.

In a preferred embodiment, the storage tank comprises a first tank body and a second tank body, and the first tank body is in communication with the second tank body; and the first tank body is used for replenishing the liquid to the second tank body, and the second tank body is in communication with the working layer via the pumping device to output the liquid to the working layer or receive the liquid from the working layer.

In a preferred embodiment, the second tank body comprises an exhaust hole.

In a preferred embodiment, the exhaust hole is in communication with the first tank body via an exhaust pipe, and a communication position between the exhaust pipe and the first tank body is higher than a maximum liquid level line of the first tank body.

In a preferred embodiment, a height difference is formed between the first tank body and the second tank body, so that the liquid in the first tank body flows to the second tank body under an action of gravity.

In a preferred embodiment, a liquid inlet of the pumping device is in communication with a liquid outlet of the second tank body, a liquid outlet of the pumping device is in communication with a liquid inlet of the carrier via the heat exchanger, and a liquid outlet of the carrier is in communication with a liquid inlet of the second tank body.

In a preferred embodiment, a one-way valve is disposed between the heat exchanger and the liquid inlet of the carrier.

In a preferred embodiment, a volume of the first tank body is larger than that of the second tank body.

In a preferred embodiment, characterized in that: the carrier comprises a main body configured for integrally wrapping a leg, a waist, an arm, an upper body or a whole body.

In a preferred embodiment, the main body comprises an outer layer, and the inflatable layer and the working layer are disposed on a side of the outer layer facing skin of a human body.

In a preferred embodiment, fabric of the working layer is divided into cloth base layers and thermoplastic polyurethane (TPU) layers, and the TPU layers in an upper fabric and a lower fabric of the working layer are connected by pressing at a preset distance along an extending direction of the working layer.

In a preferred embodiment, the main body has a cavity for integrally wrapping the leg, the waist, the arm, the upper body or the whole body, and a shape of the cavity is set according to a shape of different parts of a human body.

In a preferred embodiment, the main body further comprises a liquid inlet and a liquid outlet that are in communication with the working layer.

In a preferred embodiment, a first indentation is disposed in the working layer, and the first indentation is used for defining a flow channel in communication with the liquid inlet of the main body to the liquid outlet of the main body in the working layer; and the flow channel is disposed in a winding manner.

In a preferred embodiment, a second indentation is further disposed in the working layer, and a preset distance is formed between the second indentation and the first indentation at a position where the second indentation is located, so as to divide the liquid flowing through the second indentation into two parts.

In a preferred embodiment, the first indentation and the second indentation are curved press-fit lines.

In a preferred embodiment, press-fit points for reducing a volume of the flow channel are disposed in an array in the working layer.

In a preferred embodiment, sizes of intervals between the press-fit points are set according to different parts of a human body corresponding to the flow channel, the larger the intervals, the lower a flow resistance of the liquid flowing through a part of the different parts, and conversely, the smaller the intervals, the higher the flow resistance of the liquid flowing through the part of the different parts.

In a preferred embodiment, the inflatable layer is composed of a plurality of independent airbags, and each of the plurality of independent airbags respectively comprises an air hole for air intake and air exhaust.

Compared with the existing techniques, the technical solution of the present disclosure has the following advantages.

The present disclosure provides a drainage method and a drainage structure for a cold-hot compress device, and the method of delivering air to the working layer via the pumping device can accelerate drainage of the liquid medium in the working layer. In addition, the pumping device of the cold-hot compress device inherently has a function of delivering the air to the inflatable layer, which can also deliver the air to the working layer provided that only a control valve is added, so the structure is relatively simple and easy to implement.

### BRIEF DESCRIPTION OF THE DRAWING

FIG. 1 illustrates a structural diagrammatic view of a cold-hot compress device of a specific embodiment of the present disclosure;
FIG. 2 illustrates a structural diagrammatic view of a working layer of a carrier of the cold-hot compress device of the specific embodiment of the present disclosure;
FIG. 3 illustrates a structural diagrammatic view of an inflatable layer of the carrier of the cold-hot compress device of the specific embodiment of the present disclosure;
FIG. 4 illustrates a structural diagrammatic view of a first tank body and a second tank body of the cold-hot compress device of the specific embodiment of the present disclosure;
FIG. 5 illustrates a structural diagrammatic view of the first tank body and the second tank body of the cold-hot compress device of the specific embodiment of the present disclosure from another angle;
FIG. 6 illustrates a structural diagrammatic view of a housing of a main unit of the cold-hot compress device of the specific embodiment of the present disclosure;
FIG. 7 illustrates a structural diagrammatic view of an inner portion of the main unit of the cold-hot compress device of the specific embodiment of the present disclosure;
FIG. 8 illustrates a structural diagrammatic view of a connection between a pumping device and a three-way valve of the cold-hot compress device of the specific embodiment of the present disclosure;
FIG. 9 illustrates a sectional view of a pipeline structure integrally formed by combining a first circulation pipe, a communication pipe and a second circulation pipe of the cold-hot compress device of the specific embodiment of the present disclosure;
FIG. 10 illustrates a structural diagrammatic view of the pipeline structure formed by combining the first circulation pipe, the communication pipe and the second circulation pipe of the cold-hot compress device of the specific embodiment of the present disclosure when the main unit is connected to a single carrier;
FIG. 11 illustrates a structural diagrammatic view of the pipeline structure with a flow-dividing function formed by combining the first circulation pipe, the communication pipe and the second circulation pipe of the cold-hot compress device of the specific embodiment of the present disclosure when the main unit is connected to multiple carriers;
FIG. 12 illustrates a structural diagrammatic view of inflatable units of the inflatable layer of a leg binding carrier of the cold-hot compress device of the specific embodiment of the present disclosure;
FIG. 13 illustrates a structural diagrammatic view of the leg binding carrier equipped with an air valve of the cold-hot compress device of the specific embodiment of the present disclosure;
FIG. 14 illustrates a structural diagrammatic view of the leg binding carrier of the cold-hot compress device of the specific embodiment of the present disclosure when in use;
FIG. 15 illustrates a structural diagrammatic view of an arm binding carrier of the cold-hot compress device of the specific embodiment of the present disclosure when in use;
FIG. 16 illustrates a structural diagrammatic view of a waist binding carrier of the cold-hot compress device of the specific embodiment of the present disclosure when in use;
FIG. 17 illustrates a structural diagrammatic view of an upper body binding carrier of the cold-hot compress device of the specific embodiment of the present disclosure when in use;
FIG. 18 illustrates a structural diagrammatic view of a sleeping bag of the cold-hot compress device of the specific embodiment of the present disclosure when in use;
FIG. 19 illustrates a structural diagrammatic view of another sleeping bag of the cold-hot compress device of the specific embodiment of the present disclosure when in use;
FIG. 20 illustrates a diagrammatic view of a cold-hot compress device of Embodiment 9 of the present disclosure;
FIG. 21 illustrates a diagrammatic view of a pumping device delivering air to an inflatable layer of Embodiment 9 of the present disclosure;
FIG. 22 illustrates a diagrammatic view of the pumping device delivering the air to a working layer of Embodiment 9 of the present disclosure;
FIG. 23 illustrates a top view of the cold-hot compress device of Embodiment 9 of the present disclosure;
FIG. 24 illustrates a sectional view along an A-A direction in FIG. 23;
FIG. 25 illustrates a perspective view of an internal structure of the cold-hot compress device of Embodiment 9 of the present disclosure;
FIG. 26 illustrates a top view of the internal structure of the cold-hot compress device of Embodiment 9 of the present disclosure;
FIG. 27 illustrates an unfolded diagrammatic view of a carrier for wrapping an arm of Embodiment 9 of the present disclosure;
FIG. 28 illustrates a diagrammatic view of a flow channel in the carrier for wrapping the arm of Embodiment 9 of the present disclosure;
FIG. 29 illustrates an unfolded diagrammatic view of a carrier for wrapping an upper body of Embodiment 9 of the present disclosure;
FIG. 30 illustrates a diagrammatic view of a flow channel in the carrier for wrapping the upper body of Embodiment 9 of the present disclosure;
FIG. 31 illustrates an unfolded diagrammatic view of a carrier for wrapping a waist of Embodiment 9 of the present disclosure;
FIG. 32 illustrates a diagrammatic view of a flow channel in the carrier for wrapping the waist of Embodiment 9 of the present disclosure;
FIG. 33 illustrates an unfolded diagrammatic view of a carrier for wrapping a single leg of Embodiment 9 of the present disclosure;
FIG. 34 illustrates a diagrammatic view of a flow channel in the carrier for wrapping the single leg of Embodiment 9 of the present disclosure;
FIG. 35 illustrates an unfolded diagrammatic view of carriers for respectively wrapping two legs of Embodiment 9 of the present disclosure;
FIG. 36 illustrates a diagrammatic view of flow channels in the carriers for respectively wrapping the two legs of Embodiment 9 of the present disclosure;
FIG. 37 illustrates an unfolded diagrammatic view of a carrier for wrapping two legs simultaneously of Embodiment 9 of the present disclosure;
FIG. 38 illustrates a diagrammatic view of a flow channel in the carrier for wrapping the two legs simultaneously of Embodiment 9 of the present disclosure;
FIG. 39 illustrates a diagrammatic view of an inflatable layer in the carrier for wrapping the arm of Embodiment 9 of the present disclosure;
FIG. 40 illustrates an exploded view of the inflatable layer in the carrier for wrapping the arm of Embodiment 9 of the present disclosure;
FIG. 41 illustrates a diagrammatic view of an inflatable layer in the carrier for wrapping the upper body of Embodiment 9 of the present disclosure;
FIG. 42 illustrates a diagrammatic view of an inflatable layer in the carrier for wrapping the waist of Embodiment 9 of the present disclosure;
FIG. 43 illustrates a general principle diagram of the cold-hot compress device of Embodiment 9 of the present disclosure;
FIGS. 44-46 illustrate principle diagrams of the cold-hot compress device in a cooling mode, a heating mode, and a drainage mode, respectively, of Embodiment 9 of the present disclosure;
FIGS. 47-49 illustrate a general principle diagram and principle diagrams in a cooling mode and a heating mode, respectively, of the cold-hot compress device of Embodiment 10 of the present disclosure;

Reference numbers:
Carrier 1; inflatable layer 11; inflatable unit 1110; working layer 12; main body 130; connection structure 140; main unit 2; compressor 21; condenser 22; control circuit board 23; pumping device 24; radiator 25; heat dissipation fan 26; first circulation pipe 27; second circulation pipe 28; communication pipe 29; air pump 30; air valve 31; quick connector 32; display screen 33; loudspeaker 34; power supply 35; power interface 36; switch 37; first tank body 38; second tank body 39; three-way valve 40; internal circulation pipe 41; heating element 42; plate heat exchanger 43; control circuit 44; flow-restricting element 45.

### DETAILED DESCRIPTION OF THE EMBODIMENTS

In order to make the technical solution and the characteristics of the present disclosure clearer, the present disclosure will be further described in detail below with reference to the accompanying drawings and specific embodiments, it should be understood that these embodiments are merely used to illustrate the present disclosure and are not used to limit the scope of the present disclosure, various equivalent modifications of the present disclosure fall within the scope defined by the appended claims of the present application provided that they are made by those of skill in the art after reading the present disclosure.

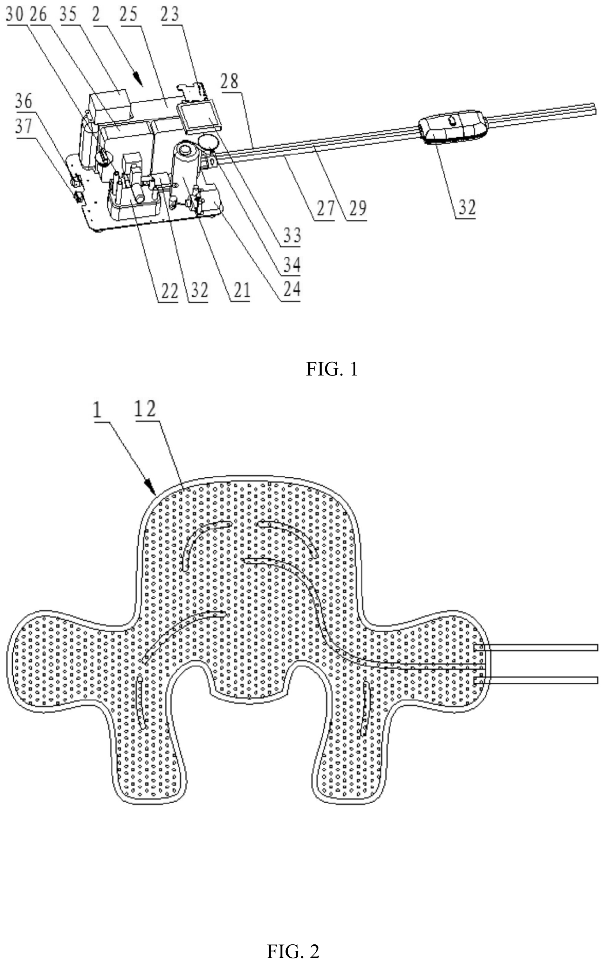

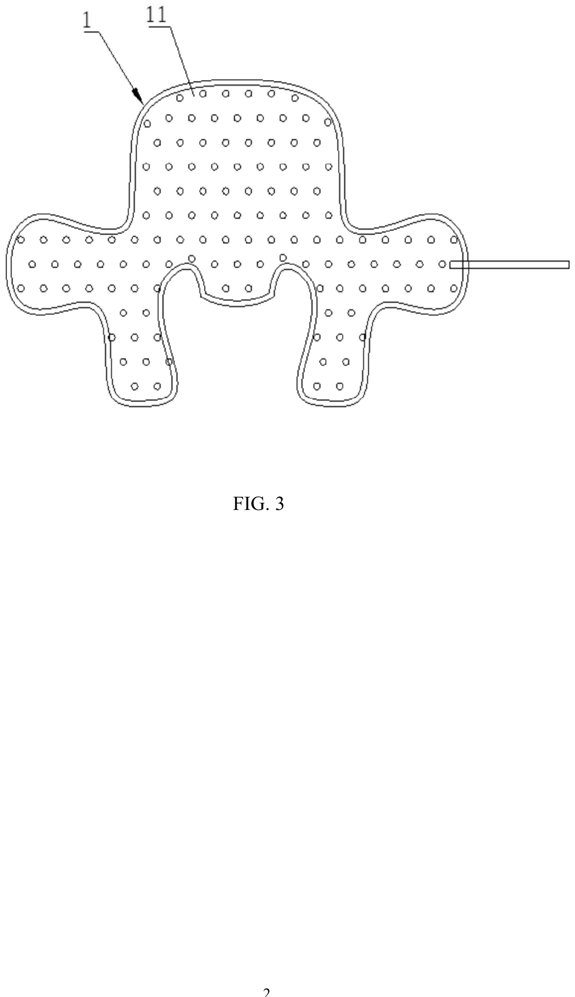

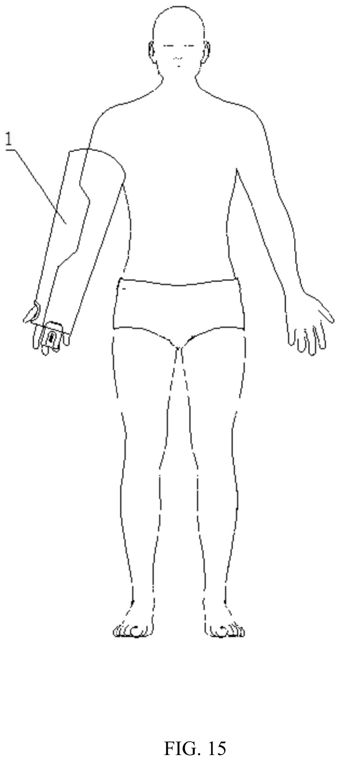

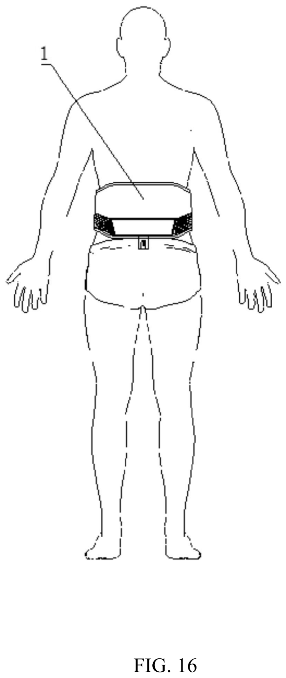

Refer to FIGS. 1-19, a cold-hot compress device comprises one or more carriers 1 and a main unit 2, a cold-hot component and a circulation component are disposed in the main unit 2. The cold-hot component comprises a compressor 21, a heat exchanger (e.g., a plate heat exchanger 43) and a flow-restricting element 45, and the compressor 21, the heat exchanger and the flow-restricting element 45 are connected in sequence; and the circulation component comprises a storage tank, a pumping device 24 and a delivery pipe, the pumping device 24 is connected to the storage tank and the one or more carriers 1 via the delivery pipe, and the delivery pipe exchanges heat with the cold-hot component.

It should be known from the preceding description that the beneficial effects of the present disclosure are as follows: through an arrangement of the circulation component, a medium is pumped by the pumping device 24 from the storage tank, exchanges heat with the cold-hot component, is then input into the one or more carriers 1 to exchange heat with a nursing part, and finally returns to the storage tank; a temperature of the medium in the delivery pipe is adjusted through cooling and heating of the cold-hot component, so that cold-hot compress function can be achieved after assembling the one or more carriers 1 on a user; and compared with the traditional semiconductor cooling and heating, the cold-hot component composed of the compressor 21, the heat exchanger and the flow-restricting element 45 has a wider application range and a larger load and can be applied to cold-hot compress operations in a large area and with a high power.

Further, a heat dissipation component is disposed in the main unit 2, the heat dissipation component comprises a radiator 25 and a heat dissipation fan 26, the radiator 25 is disposed on a side of the main unit 2, and the heat dissipation fan 26 is disposed on a side of the radiator 25.

It should be known from the preceding description that working heat in the main unit 2 can be initiatively and quickly discharged using a combination of the heat dissipation fan 26 and the radiator 25 to avoid affecting the temperature of the medium in the delivery pipe.

Further, the storage tank comprises a first tank body 38 and a second tank body 39, the first tank body 38 is in communication with the second tank body 39, and the second tank body 39 is in communication with the pumping device 24 and the one or more carriers 1.

It should be known from the preceding description that when the main unit 2 is in communication with a single one of the one or more carriers 1, a certain amount of the medium stored in the second tank body 39 can be used to provide sufficient supply for circulation; when the main unit 2 is in communication with multiple of the one or more carriers 1, the medium in the second tank body 39 cannot, by itself, provide sufficient supply for circulation, and at this time, the first tank body 38 replenishes the medium to the second tank body 39 for adjustment and improvement. At the same time, when the second tank body 39 is used for small circulation, rapid temperature rise and temperature fall of the single one of the one or more carriers 1 can be realized.

Further, the cold-hot component further comprises a heating element 42, and the heating element is used for heating the medium in the delivery pipe.

It should be known from the preceding description that the heating element 42 is used to achieve a heating function of the cold-hot component, and optionally, the heating element can be an electric heating wire. A combination of the compressor 21, the condenser 22, an expansion valve and the evaporator achieves a cooling function of the cold-hot component; and the heating function and the cooling function are respectively realized by controlling the two components.

Further, a channel of the delivery pipe comprises an internal circulation pipe 41, a first circulation pipe 27 and a second circulation pipe 28, the internal circulation pipe 41 is disposed in the main unit 2, and the one or more carriers 1, the pumping device 24 and the storage tank are all connected via the internal circulation pipe 41; and the first circulation pipe 27 and the second circulation pipe 28 are connected to the internal circulation pipe and the one or more carriers 1.

It should be known from the preceding description that the one or more carriers 1, the pumping device 24 and the storage tank are connected via the internal circulation pipe 41, and the first circulation pipe 27 and the second circulation pipe 28 are used as connecting pipes between the internal circulation pipe 41 and the one or more carriers 1 to output and input the medium in the one or more carriers 1.

Further, the delivery pipe further comprises a communication pipe 29; an air pump 30 is disposed in the main unit 2, one end of the air pump 30 is connected to one or more air valves 31, and an inflatable layer 11 is disposed on the one or more carriers 1; and the air pump 30, the one or more air valves 31 and the inflatable layer 11 of the one or more carriers 1 are all connected via the communication pipe 29.

It should be known from the preceding description that filling air is output by the air pump 30 and the one or more air valves 31 and is filled into the inflatable layer 11 of the one or more carriers 1 via the communication pipe 29 to enable the one or more carriers 1 to be inflated so as to be closely fitting with the nursing part.

Further, a three-way valve 40 is disposed in the main unit 2, the pumping device 24 is an air-liquid dual-purpose pump, a first end of the three-way valve 40 is connected to the storage tank, a second end of the three-way valve 40 is connected to atmosphere, and a third end of the three-way valve 40 is connected to the pumping device 24.

It should be known from the preceding description that through a design of the three-way valve 40 in the main unit 2 and an application of the air-liquid dual-purpose pump, when the medium in the one or more carriers 1 needs to be discharged, this can be achieved by pumping air with the pumping device 24 through on-off control of the three-way valve 40.

Further, an installation height of the first tank body 38 is higher than an installation height of the second tank body 39.

It should be known from the preceding description that through a height setting of the first tank body 38 and the second tank body 39, when the medium in the second tank body 39 is insufficient to support a circulation supply, the medium in the first tank body 38 can quickly flow into the second tank body 39 under the action of its own weight.

Further, the inflatable layer 11 comprises a plurality of inflatable units 1110, each of the inflatable units 1110 is connected to one of the one or more air valves, and multiple of the one or more air valves 31 are connected to the air pump 30.

It should be known from the preceding description that through a design of the plurality of inflatable units 1110 and the one or more air valves 31, a zonal step-by-step pneumatic compression of a human body part in a coverage area of the one or more carriers 1 can be achieved, so that blood of the human body part can flow back quickly, a hyperemia state of the part after exercise can be alleviated, fatigue after exercise can be reduced, and repair of the part can be accelerated.

Further, the one or more air valves 31 are disposed on the one or more carriers 1.

It should be known from the preceding description that by arranging the one or more air valves 31 on the one or more carriers 1, an occupied installation space on the main unit 2 can be reduced, and sufficient installation space can be reserved in the main unit 2 for configuring other components.

Further, a carrier 1 comprises a connection structure 140 and a main body 130 configured to be used for integrally wrapping a leg, a waist, an arm, an upper body or a whole body, and the connection structure 140 is disposed on the main body 130.

It should be known from the preceding description that the carrier 1 adopts an integral wrapping design for parts to conveniently and quickly wrap the leg, the waist, the arm, the upper body and the whole body, so as to achieve an integral cold-hot compress treatment; and compared with a wearing style with multiple carriers 1 in divided zones in the existing techniques, the one or more carriers 1 with this design can be worn more conveniently. The one or more carriers 1 for integrally wrapping are able to be worn without guidance of professional personnel in a wearing process. Specifically, the connection structure 140 can adopt a quick disassembly and assembly design such as a zipper structure or a hook and loop fastener structure.

Referring to FIGS. 1-19, Embodiment 1 of the present disclosure is as follows:
A cold-hot compress device comprises a carrier 1 and a main unit 2.

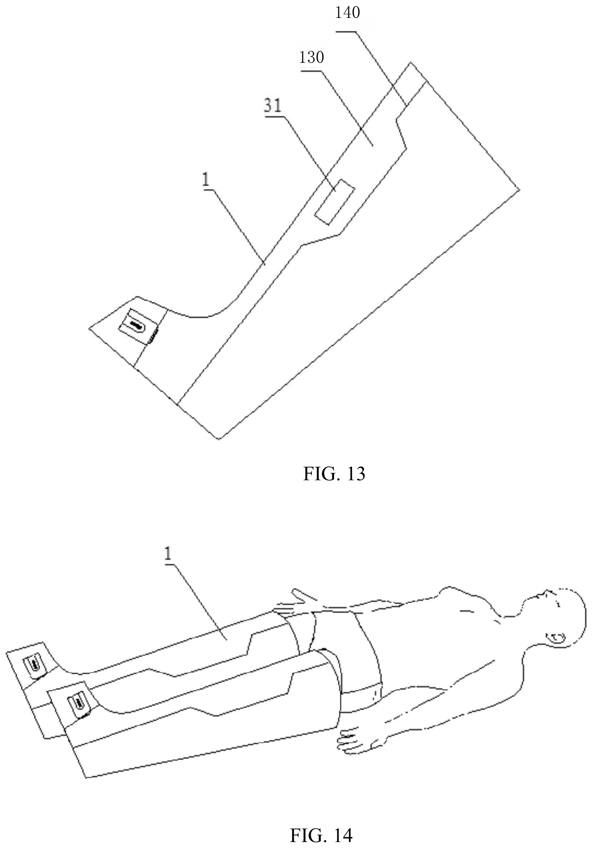

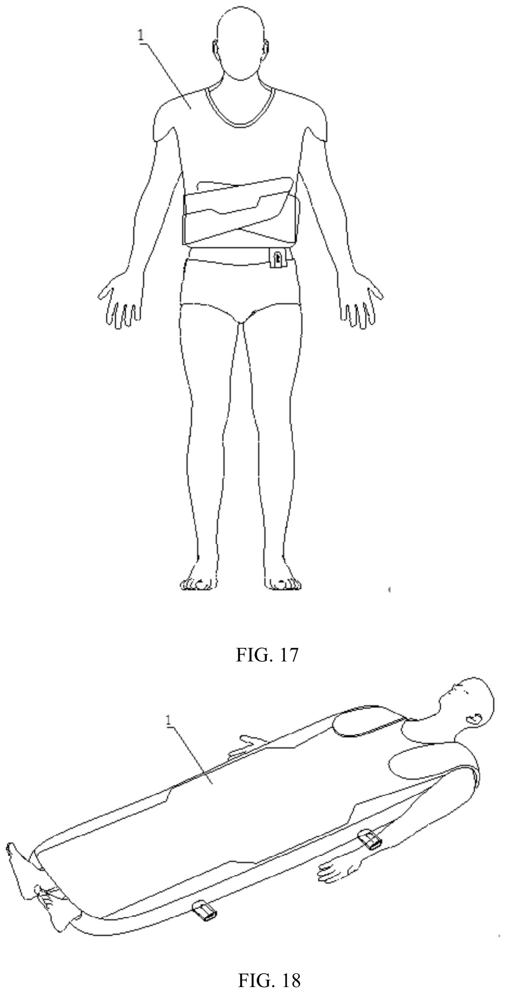

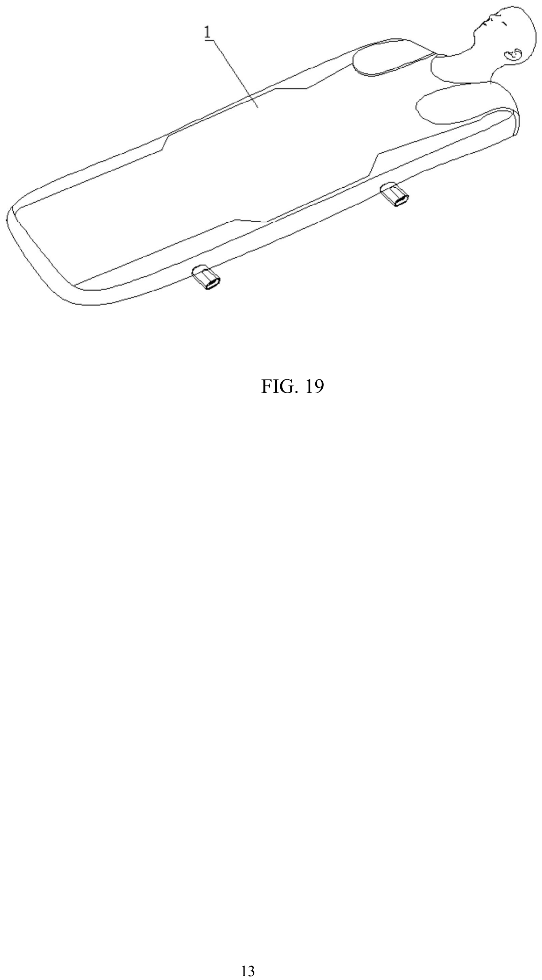

The carrier 1 comprises an inflatable layer 11 and a working layer 12, after the inflatable layer 11 is filled with a medium, the carrier 1 can be closely attached to a nursing part, and the working layer 12 is filled with a cooled or heated medium so as to perform cold-hot compress on the attached nursing part. The inflatable layer 11 and the working layer 12 both comprise a plurality of concave structures, therefore, after filling with the medium, the inflatable layer 11 and the working layer 12 can contact the nursing part with multiple points, fit more closely, and achieve a certain squeezing and massaging effect. Specifically, as shown in FIGS. 14-19, the carrier 1 comprises a component for wrapping a leg, a waist, an arm, a shoulder and a neck, an upper body and the whole body, and types of the carrier 1 include leg binding carriers, waist binding carriers, arm binding carriers, upper-body binding carriers (such as a wearable upper garment-shaped carrier for nursing and treating a chest, a back, shoulders and a neck), and sleeping bags (which can perform cold-hot compress nursing on the whole body), which can be replaced and used according to specific needs; and specifically, FIGS. 18 and 19 illustrate two styles of the sleeping bags, in the sleeping bag corresponding to FIG. 18, arms and feet extend out of the sleeping bag, the sleeping bag corresponding to FIG. 19 can wrap the arms and the feet, and only a head of a user is exposed, so as to achieve more comprehensive cold-hot compress nursing.

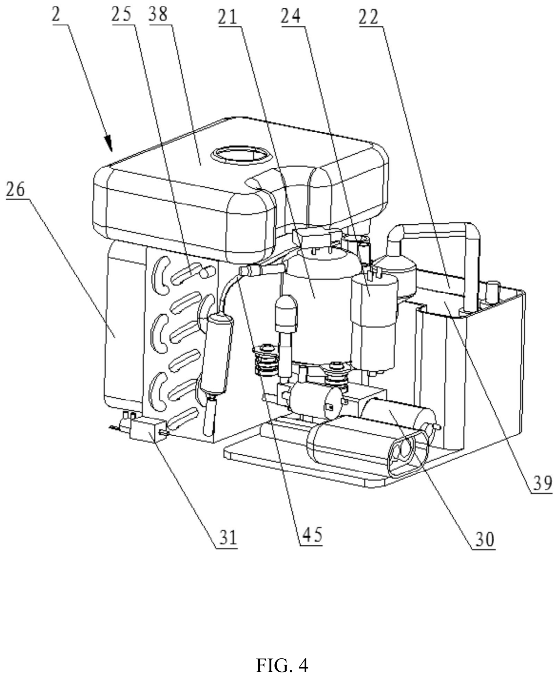

A cold-hot component, a circulation component, a heat dissipation component, a communication pipe 29, an air pump 30 and one or more air valves 31 are disposed in the main unit 2. The cold-hot component comprises a compressor 21, a heat exchanger and a flow-restricting element 45, in this embodiment, the heat exchanger comprises a condenser 22 and an evaporator, and the flow-restricting element 45 is an expansion valve. Optionally, as shown in FIG. 4, the flow-restricting element 45 can also be a capillary tube, and the heat exchanger can also be a plate heat exchanger 43. The compressor 21, the condenser 22, the expansion valve and the evaporator are in communication in sequence; the circulation component comprises a storage tank, a pumping device 24 and a delivery pipe, the pumping device 24 is connected to the storage tank and the carrier 1 via the delivery pipe, and the delivery pipe exchanges heat with the cold-hot component. Specifically, a pressure relief valve is further disposed on the pumping device 24.

Specifically, the main unit 2 comprises a housing and a mounting plate, the mounting plate is disposed on a bottom of the housing, and the cold-hot component, the circulation component and the heat dissipation component are all locked to the mounting plate. As shown in FIG. 1, the housing is a cuboid or a cube as a whole, and the storage tank is disposed on one end of an inner portion of the housing along a length direction, and the heat dissipation component and the cold-hot component are disposed on another end of the inner portion of the housing along the length direction. Through an arrangement design of the storage tank, the heat dissipation component and the cold-hot component, the storage tank does not interfere with heat dissipation of the cold-hot component, and sufficient space is ensured for discharging working heat of the main unit 2. The heat dissipation component and the cold-hot component are disposed side by side along a width direction of the housing. The heat dissipation component comprises a radiator 25 and a heat dissipation fan 26, the radiator 25 is disposed on a side surface of the housing, and the heat dissipation fan 26 is disposed between the radiator 25 and the cold-hot component.

A power supply 35 and a control circuit board 23 electrically connected to the power supply 35, the cold-hot component, the circulation component and the heat dissipation component are disposed in the main unit 2, a display screen 33 and a loudspeaker 34 are disposed on an upper side of the main unit 2, and the display screen 33 and the loudspeaker 34 are also connected to the control circuit board 23 for observing an operation state and prompting an alarm. A power interface 36 and a switch 37 are disposed on a rear portion of the main unit 2.

The delivery pipe comprises an internal circulation pipe 41, a first circulation pipe 27 and a second circulation pipe 28, the internal circulation pipe 41 is disposed in the main unit 2, and the carrier 1, the pumping device 24 and the storage tank are all connected together via the internal circulation pipe 41; the first circulation pipe 27 and the second circulation pipe 28 are connected to the internal circulation pipe 41 and the carrier 1; and the first circulation pipe 27 and the second circulation pipe 28 are double-layer pipes, each of the double-layer pipes comprises an inner layer and an outer layer, the inner layer is hollow and is used for the medium to pass through, and the outer layer is filled with substances, such as air, which can prevent the inner layer from thermal radiation. A design of the double-layer pipes can achieve heat preservation of the medium in the first circulation pipe 27 and the second circulation pipe 28.

The air pump 30 is connected to the one or more air valves 31, the communication pipe 29 and the inflatable layer 11 of the carrier 1, and the air is filled into the inflatable layer 11 of the carrier 1 via the air pump 30, so that the carrier 1 can be closely attached to the nursing part.

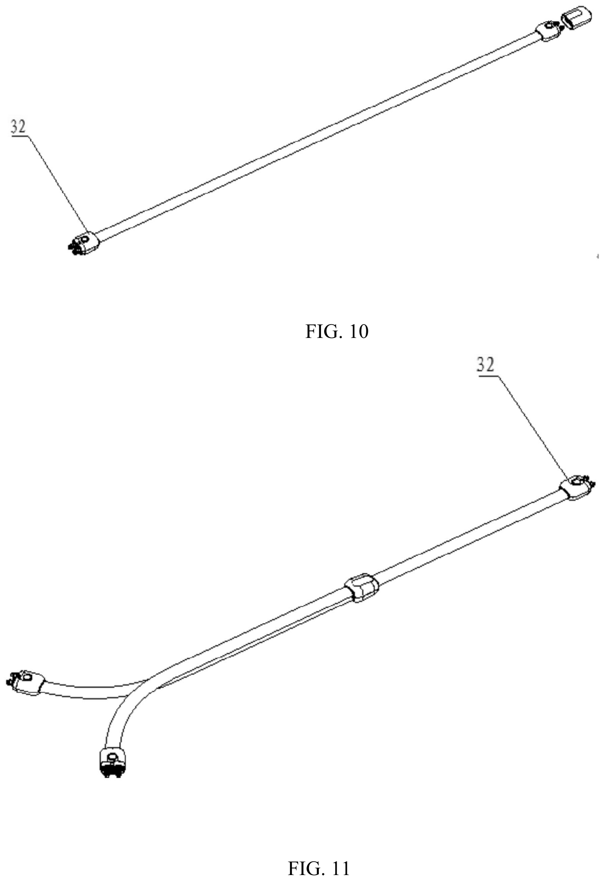

As shown in FIG. 10, the first circulation pipe 27, the communication pipe 29 and the second circulation pipe 28 are disposed side by side and connected integrally to be assembled conveniently and uniformly. Quick connectors 32 are disposed on middle portions of all of the first circulation pipe 27, the second circulation pipe 28 and the communication pipe 29, so that extension or external connection of multiple groups of carriers 1 can be implemented as needed. Specifically, as shown in FIG. 11, when a pipeline structure formed by combining the first circulation pipe 27, the second circulation pipe 28 and the communication pipe 29 is connected to multiple groups of carriers 1, a flow-dividing connector is used for flow dividing configuration.

### Embodiment 2:

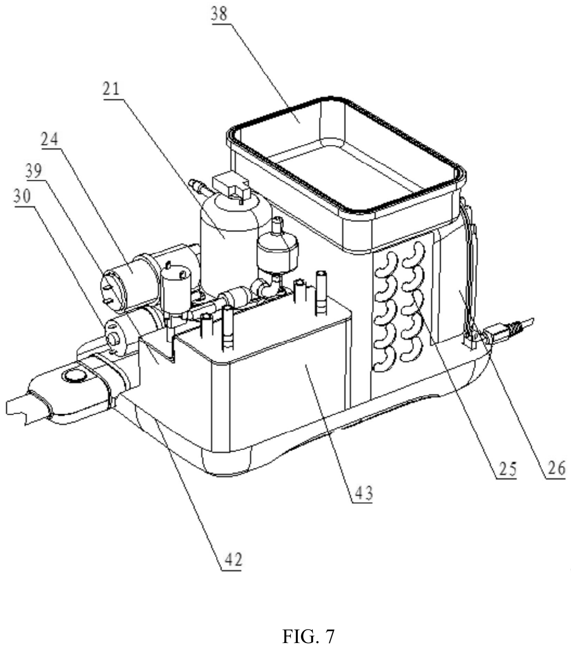

Embodiment 2 differs from Embodiment 1 in that: the cold-hot component further comprises an electric heating wire, the electric heating wire is used as a component for heating the medium in the delivery pipe, and a combination of the compressor 21, the condenser 22, the expansion valve, and the evaporator is used for cooling the medium in the delivery pipe. In other embodiments, the cold-hot component further comprises a heating plate, the heating plate is used as the heating element 42, and the heating plate can be disposed on a side of the delivery pipe, on a side of the storage tank or on other parts capable of conducting heat to the medium according to an installation space inside of the main unit. In FIG. 7, the heating element 42 is disposed on a bottom of the storage tank.

### Embodiment 3:

Embodiment 3 differs from Embodiment 1 in that: a three-way valve 40 is disposed in the main unit 2, and the air pump 30, the communication pipe 29 and the internal circulation pipe 41 are connected via the three-way valve 40. Through an arrangement of the three-way valve 40, the air pump 30 can pump the air into the carrier 1 so as to discharge and empty the medium in the carrier 1.

### Embodiment 4:

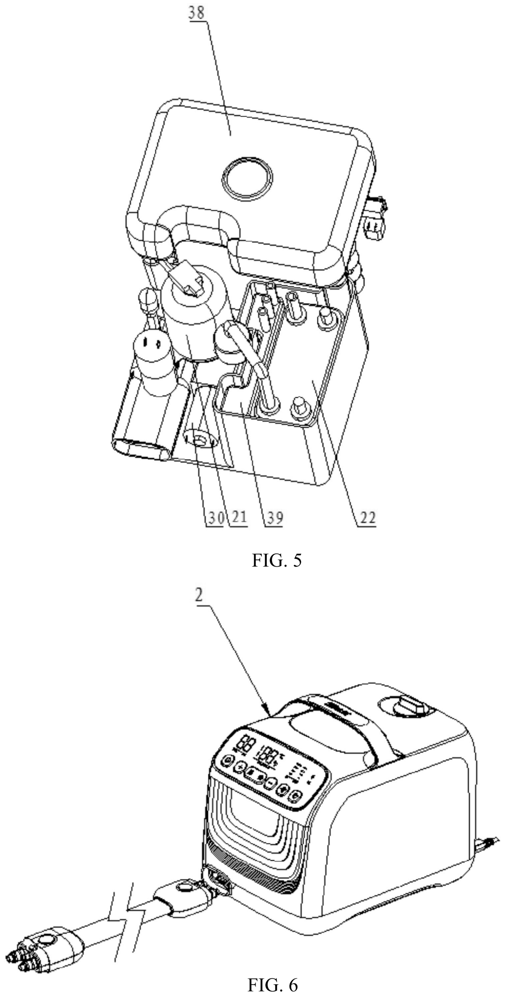

Embodiment 4 differs from Embodiment 1 in that: as shown in FIGS. 4 and 5, the heat dissipation fan 26, the radiator 25 and the cold-hot component are disposed in sequence along a length direction of the housing.

### Embodiment 5:

Embodiment 5 differs from Embodiment 1 in that, as shown in FIGS. 4 and 5, the storage tank comprises a first tank body 38 and a second tank body 39, the first tank body 38 is in communication with the second tank body 39, and the second tank body 39 is in communication with the pumping device 24 and the carrier 1. An installation height of the first tank body 38 is higher than an installation height of the second tank body 39. Specifically, a control valve is connected to the first tank body 38 and the second tank body 39, a water level detection device is disposed in the second tank body 39, and the water level detection device is electrically connected to the control valve. In addition, the principle of communicating vessels can also be adopted, and automatic replenishment is achieved by utilizing a water level difference between the first tank body 38 and the second tank body 39 when water in the second tank body 39 is insufficient.

### Embodiment 6:

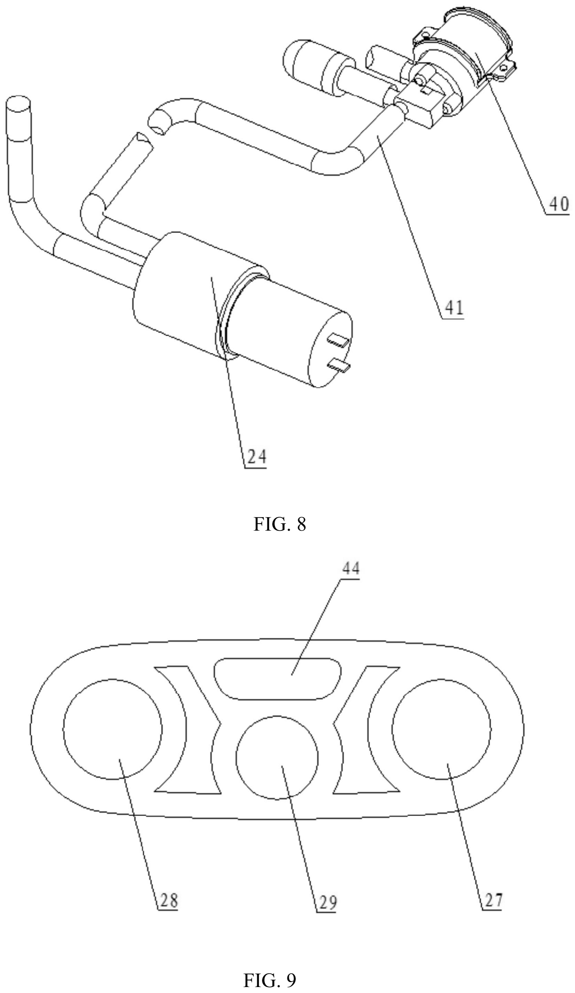

Embodiment 6 differs from Embodiment 3 in that: as shown in FIG. 8, the pumping device 24 is an air-liquid dual-purpose pump, a first end of the three-way valve 40 is connected to the storage tank, a second end of the three-way valve 40 is connected to the atmosphere, and a third end of the three-way valve 40 is connected to the pumping device 24. Through a design of the air-liquid dual-purpose pump combined with a design of the three-way valve 40, the air and the medium inside of the carrier 1 can be pumped, and an installation space inside of the main unit 2 occupied by multiple pumping devices can be reduced.

### Embodiment 7:

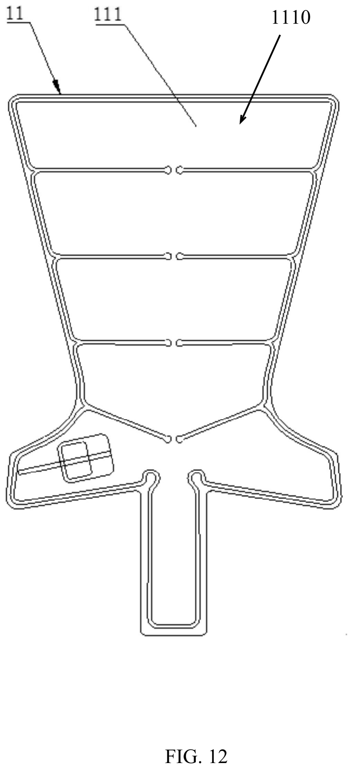

Embodiment 7 differs from Embodiment 1 in that: as shown in FIG. 12, the inflatable layer 11 comprises a plurality of inflatable units 1110, each of the inflatable units 1110 is connected to one of the one or more air valves 31, and multiple of the one or more air valves 31 are connected to the air pump 30; and through a design of the plurality of inflatable units 1110 and the one or more air valves 31, a zonal step-by-step pneumatic compression of a human body part in a coverage area of the carrier 1 can be achieved, so that blood of the human body part can flow back quickly, a hyperemia state of the part after exercise can be alleviated, fatigue after exercise can be reduced, and repair of the part can be accelerated. Multi-functional uses, such as cold compress, hot compress, inflatable binding to improve a fitting effect, and the zonal step-by-step pneumatic compression for a treatment of hyperemic parts, can be achieved using the carrier 1 in this embodiment.

In other embodiments, as shown in FIG. 13, the one or more air valves 31 are disposed on the carrier 1; and by arranging the one or more air valves 31 on the carrier 1, an occupied installation space on the main unit 2 can be reduced, and a sufficient installation space can be reserved in the main unit 2 for configuring other components. As shown in FIG. 9, correspondingly, a control circuit 44 connected to the one or more air valves 31 is disposed in a pipeline structure formed by arranging and integrally connecting the first circulation pipe 27, the communication pipe 29 and the second circulation pipe 28 side by side .

### Embodiment 8:

Embodiment 8 differs from Embodiment 1 in that: according to different matched functions of the carrier 1, the device can have various styles. When the main unit 2 is matched with the carrier without the treatment of the hyperemic parts, the one or more air valves 31 are disposed in the main unit 2 with this style, and the one or more air valves 31 are not disposed on the carrier 1; when the main unit 2 is only matched with the carrier 1 with the treatment of the hyperemic parts, the one or more air valves 31 are not disposed in the main unit 2 with this style, and the one or more air valves 31 are disposed on the carrier 1; when the main unit 2 can be matched with both of the carrier 1 without treatment of the hyperemic parts and the carrier 1 with the treatment of the hyperemic parts, a main air valve is disposed in the main unit 2 with this style, and the main air valve receives signals from the control circuit board 23 to switch on-off operations; in addition, the main air valve can also be in a normally open state, and the on-off operations are controlled by another control component; and a secondary air valve is disposed on the carrier 1 with the treatment of the hyperemic parts, and no air valves 31 are disposed on the carrier 1 without the treatment of the hyperemic parts.

### Embodiment 9

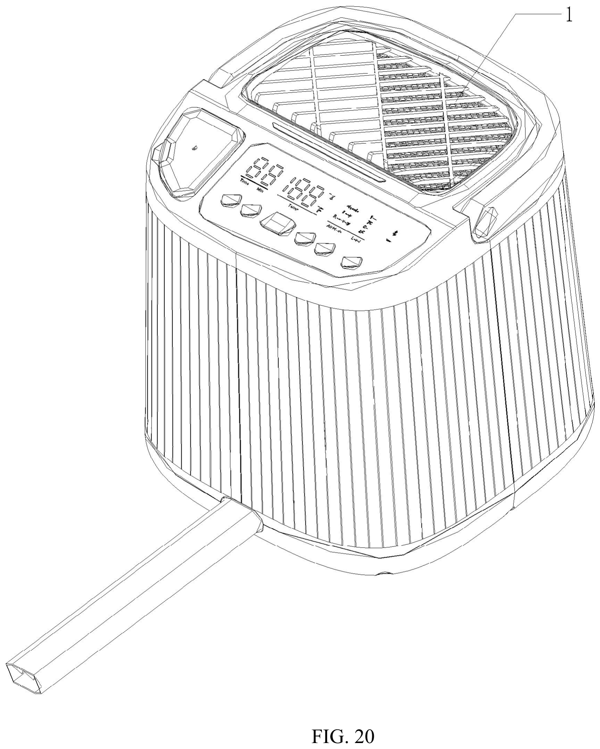

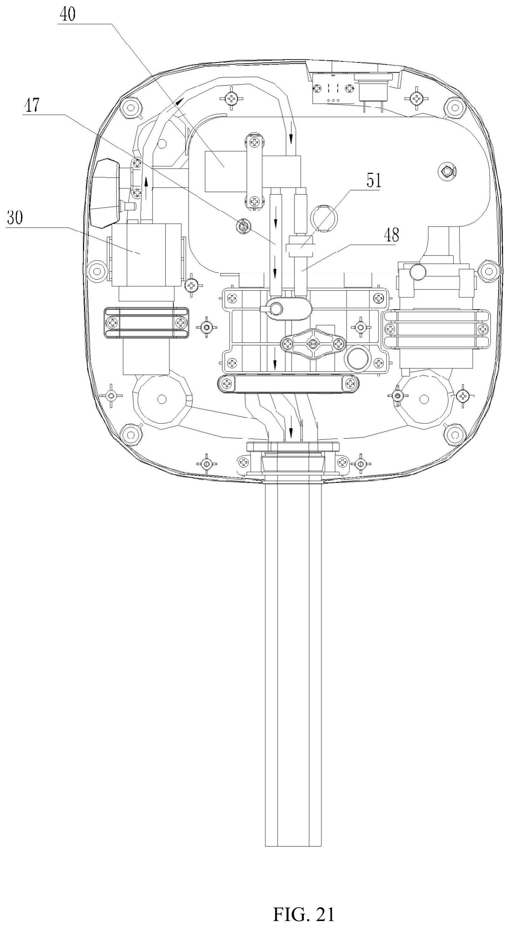

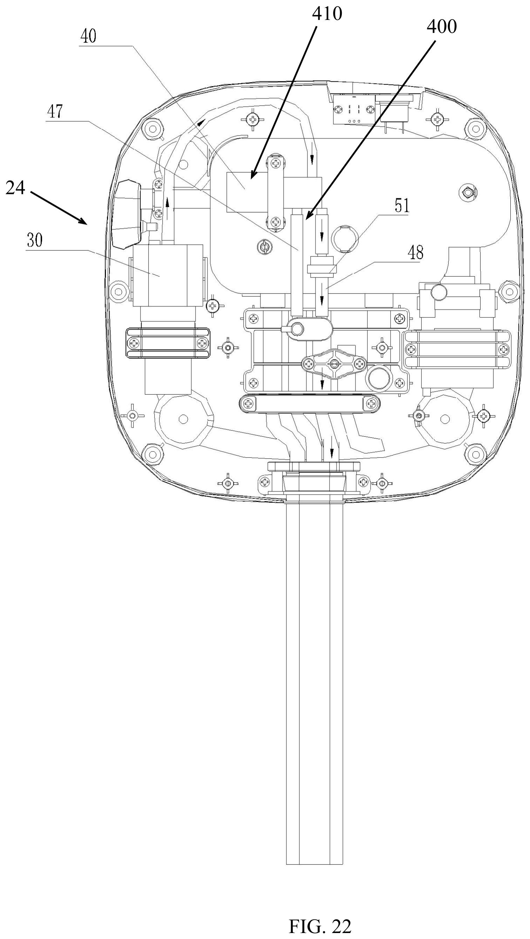

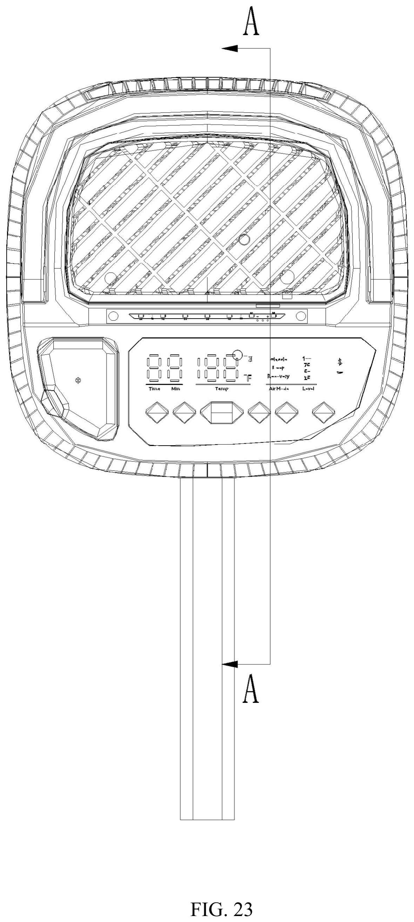

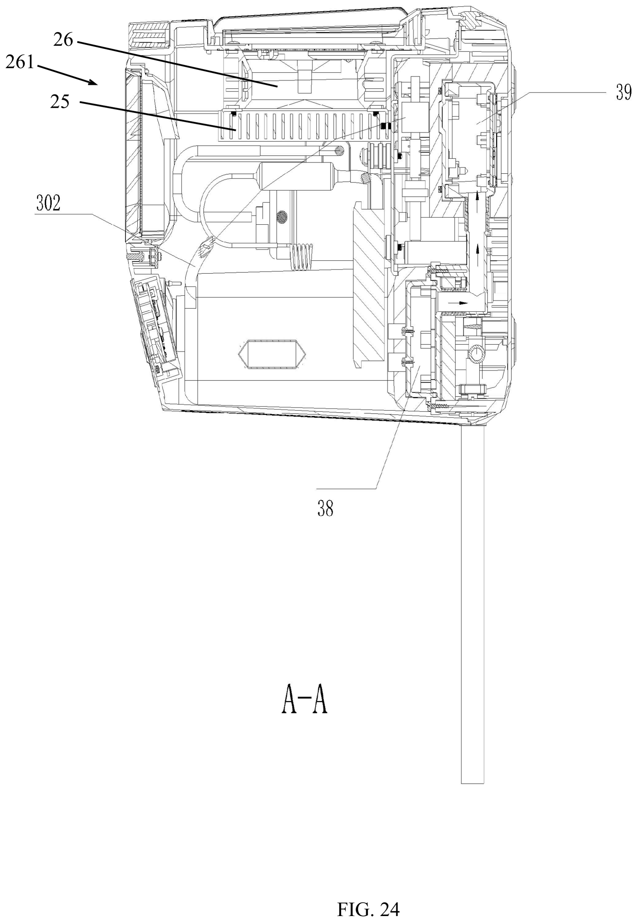

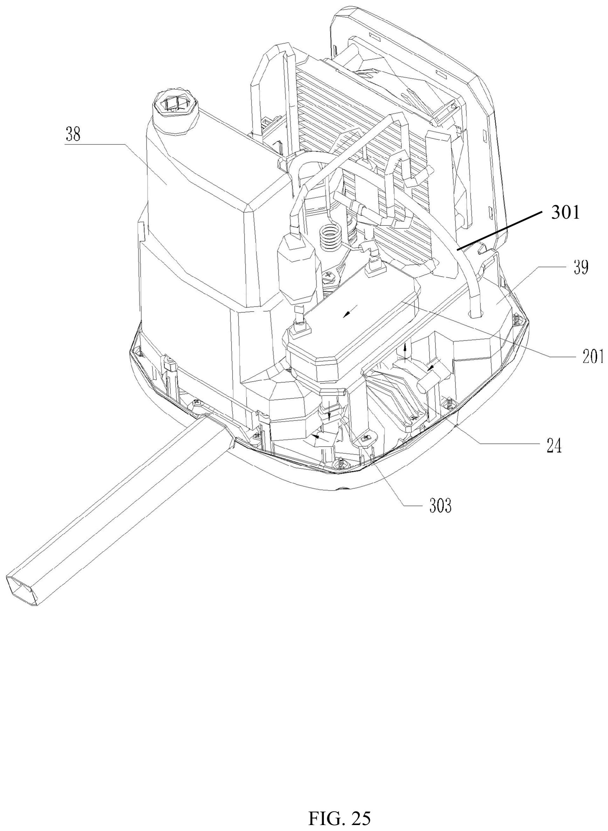

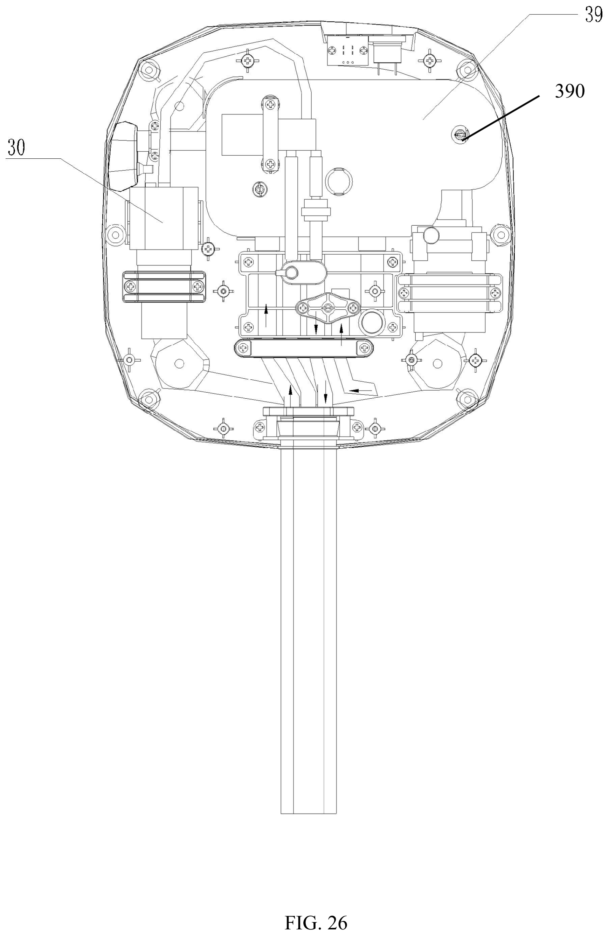

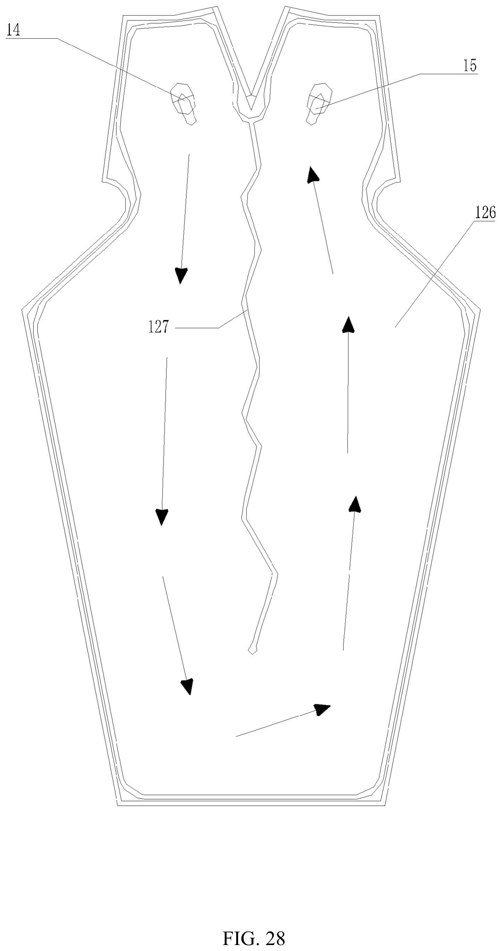

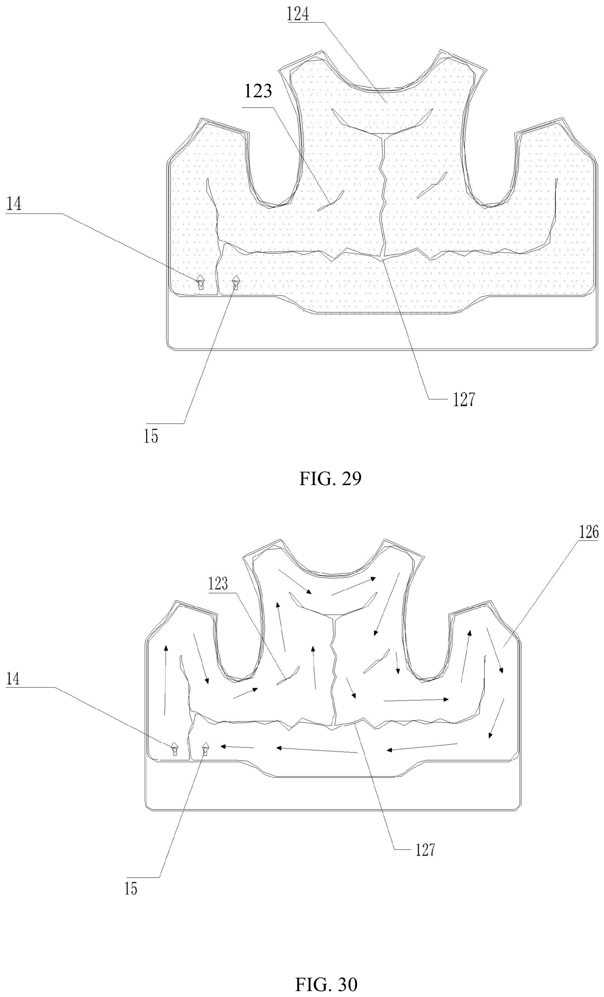

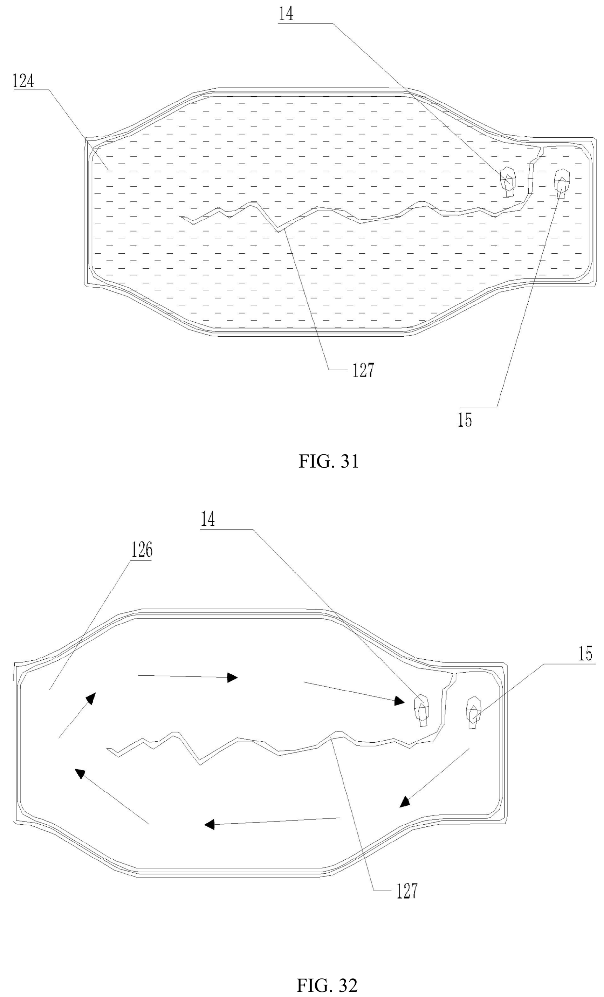

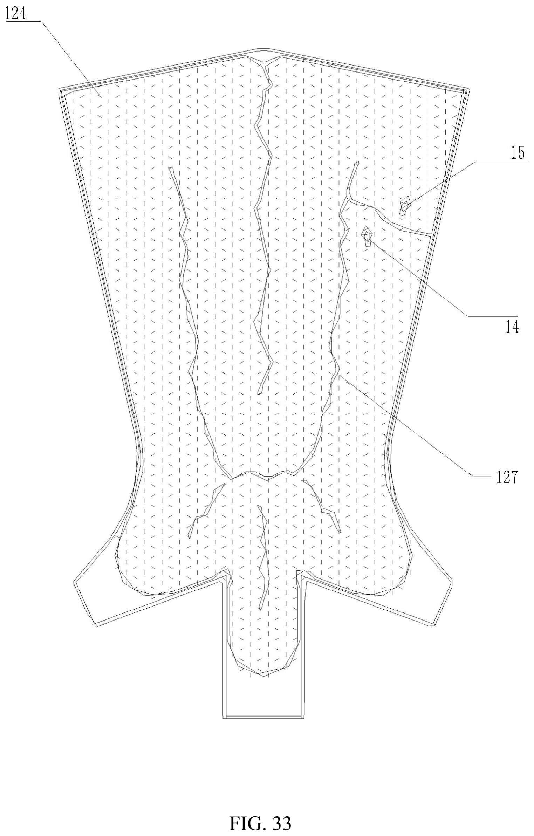

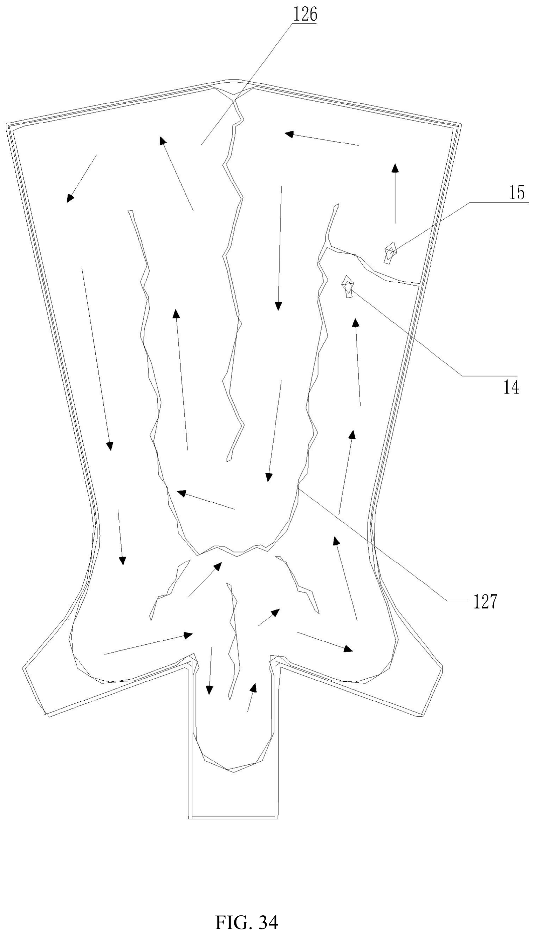

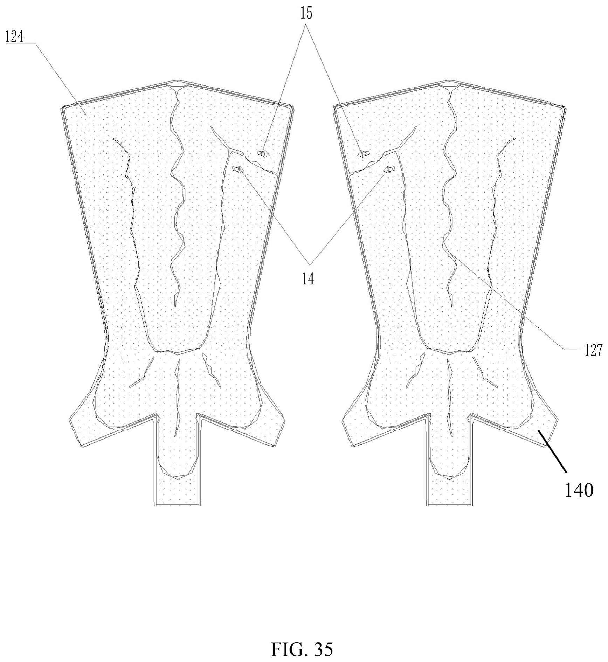

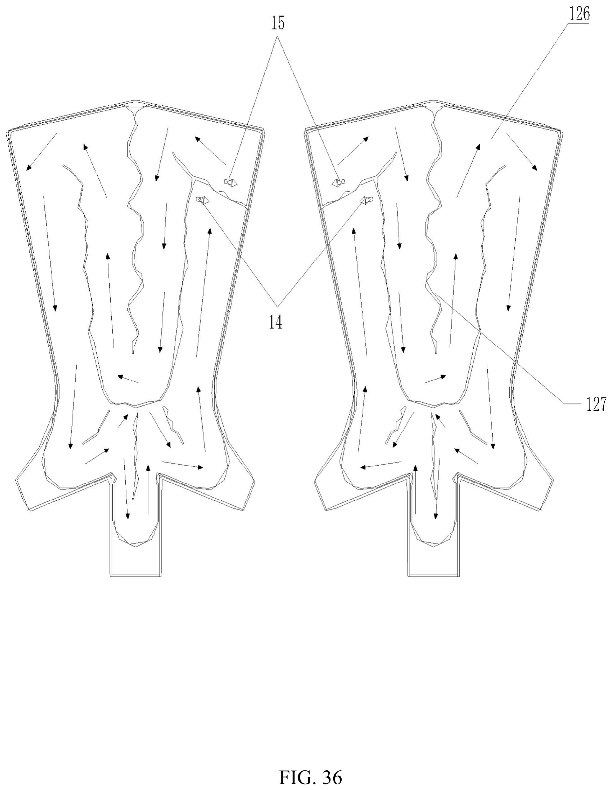

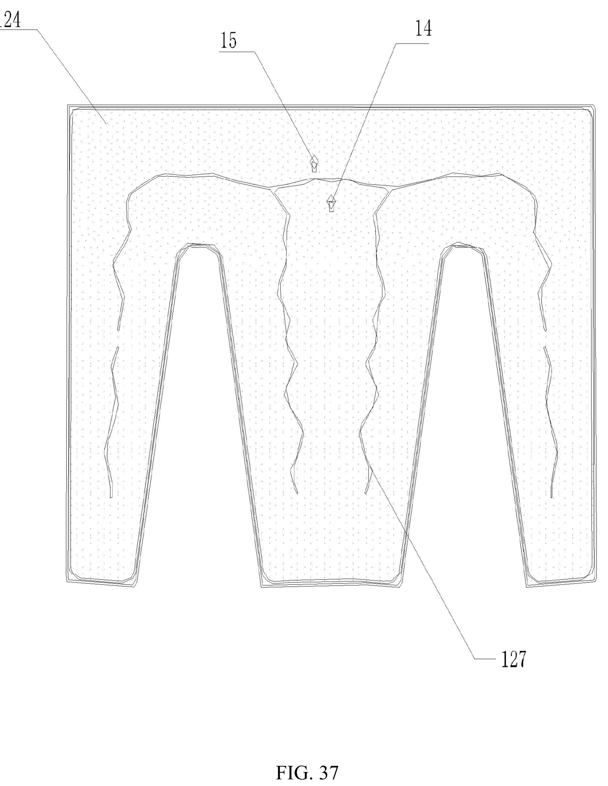

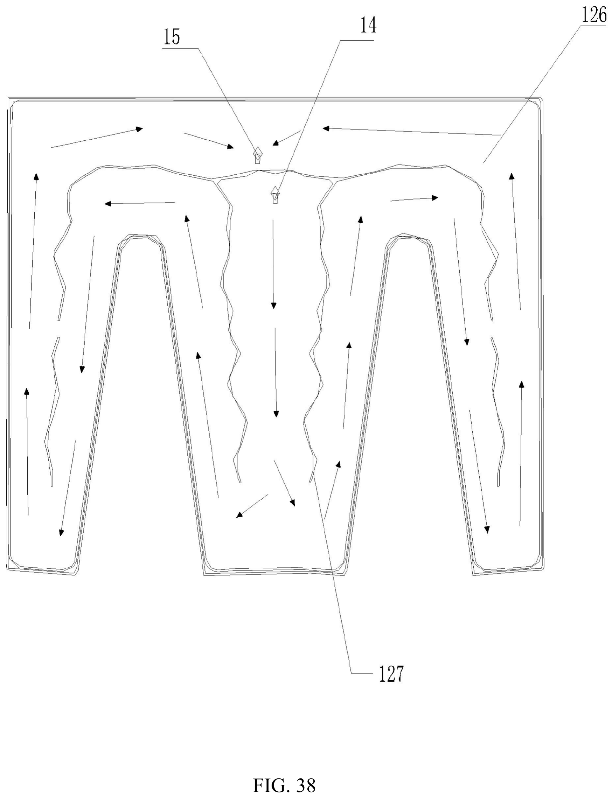

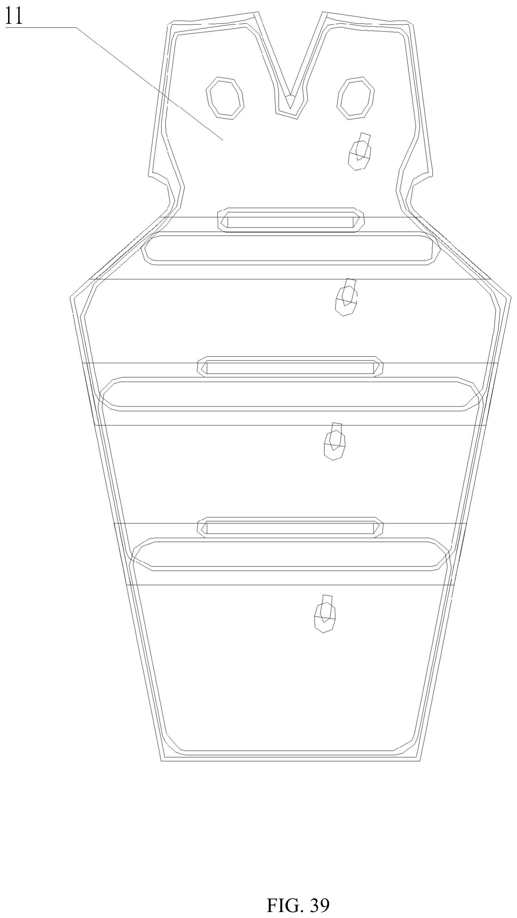

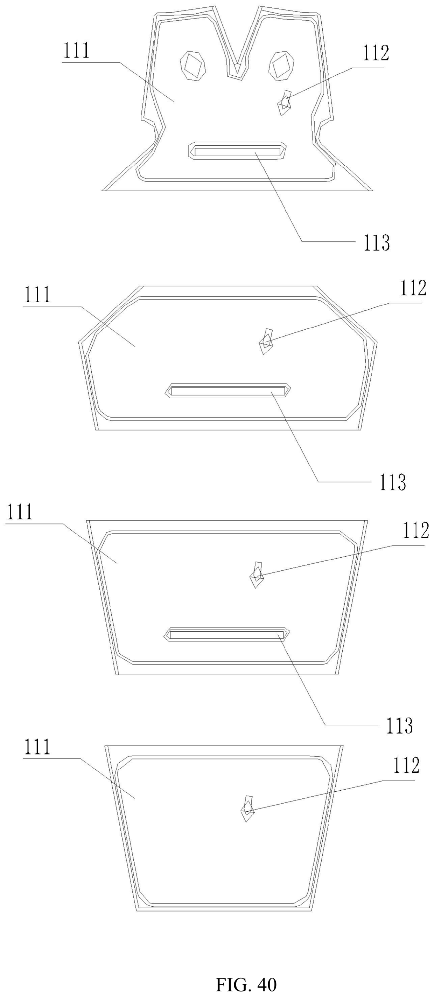

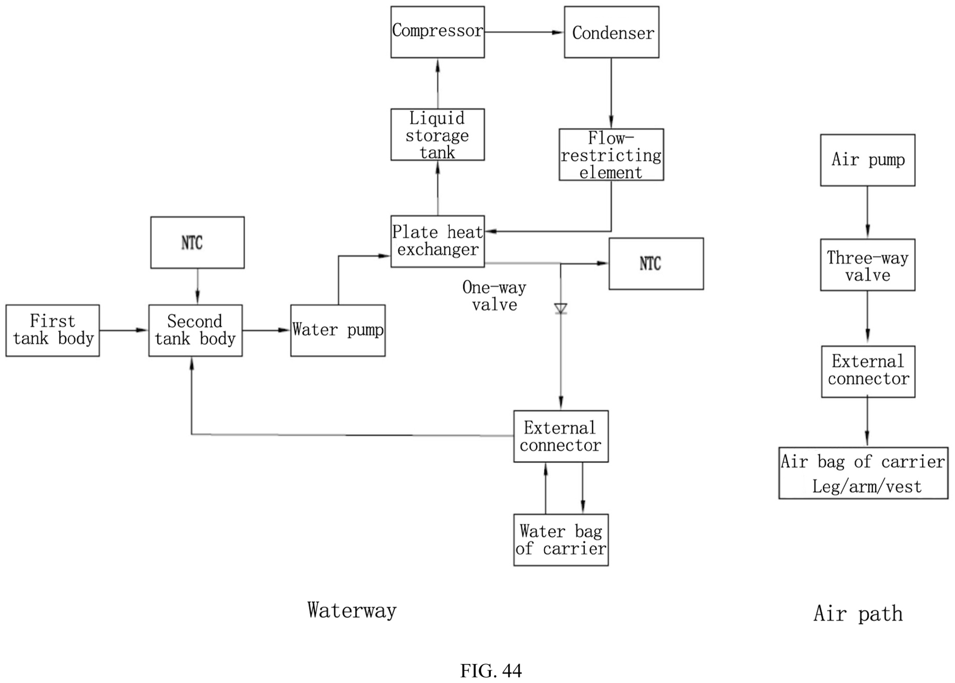

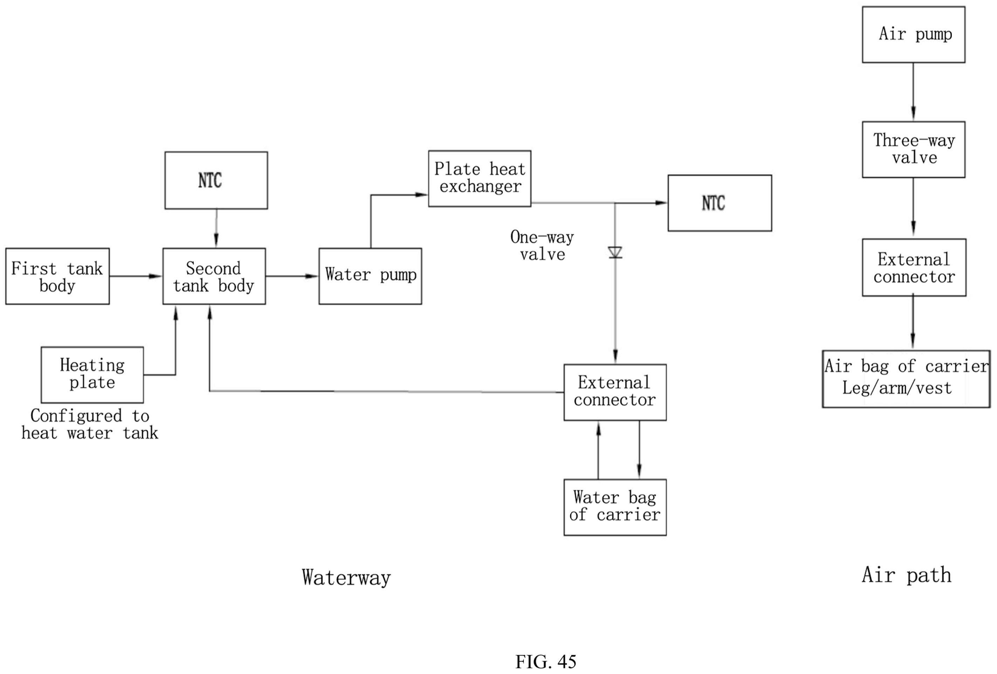

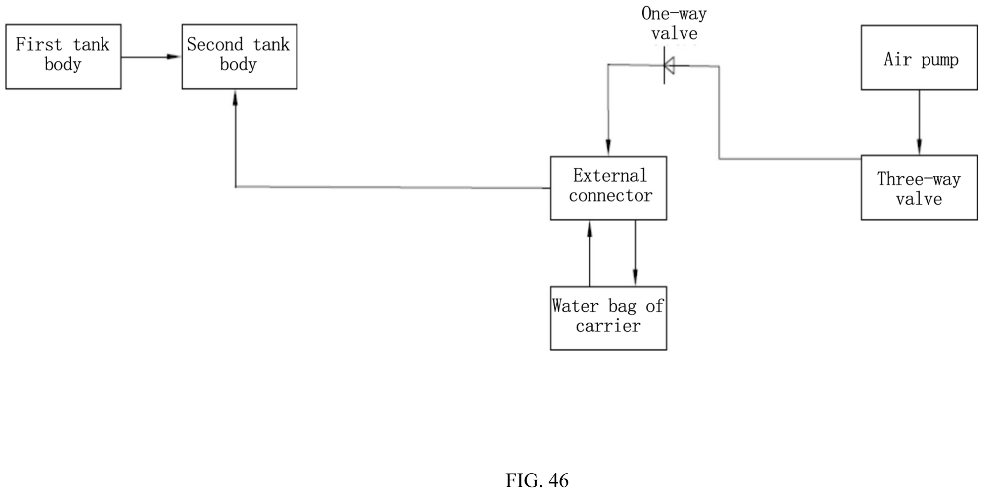

Referring to FIGS. 20-46, in this embodiment, delivery pipes 400 are specifically divided into an air path delivery pipe 47 and a waterway delivery pipe 48. An air pump 30 and a water pump 201 in a pumping device 24 are respectively connected to an inflatable layer 11 and a working layer 12 via the air path delivery pipe 47 and the waterway delivery pipe 48. The air pump 30 in the pumping device 24 is further connected to the waterway delivery pipe 48 via a control valve 410, so as to deliver air to the working layer 12 via the waterway delivery pipe 48 when the control valve 410 is opened.

This embodiment is the same as Embodiment 6 in that the control valve 410 is a three-way valve 40 with one inlet and two outlets. The inlet of the three-way valve 40 is in communication with an air outlet of the air pump 30, a first outlet of the two outlets is connected to the air path delivery pipe 47, and a second outlet of the two outlets is connected to the waterway delivery pipe 48.

The inlet of the three-way valve 40 and the air path delivery pipe 47 form a normally open line, and the inlet of the three-way valve 40 and the waterway delivery pipe 48 form a normally closed line.

When a cold-hot compress component (i.e., a cold-hot compress device) works, the normally open line of the three-way valve 40 is conducted (i.e., opened), and the normally closed line is closed. Air output by the air pump 30 is delivered to the inflatable layer 11 through the normally open line to enable the inflatable layer 11 to expand, so as to achieve a function of inflating the air into the carrier 1 by the air pump 30.

After an operation of the cold-hot compress component is completed, the normally closed line of the three-way valve 40 is conducted (i.e., opened), and the normally open line is closed. The air output by the air pump 30 is delivered to the working layer 12 through the normally closed line to be blown into the working layer 12, so that liquid in the working layer 12 is blown out.

In addition, in this embodiment, a one-way valve 51 is disposed between the second outlet of the three-way valve 40 and the waterway delivery pipe 48 to prevent water in the waterway delivery pipe 48 from flowing to the air pump 30 when the normally closed line is opened.

The storage tank 300 is used for storing the liquid configured to be delivered to the carrier 1 and comprises a first tank body 38 and a second tank body 39, and the first tank body 38 is in communication with the second tank body 39. The first tank body 38 is used for replenishing the liquid to the second tank body 39, and the second tank body 39 is in communication with the carrier 1 via the water pump 201 in the pumping device 24 to output the liquid to the carrier 1 or receive the liquid output from the carrier 1. Specifically, a liquid inlet of the water pump 201 is in communication with a liquid outlet of the second tank body 39, a liquid outlet of the water pump 201 is in communication with a liquid inlet of the carrier 1 via a heat exchanger 43 (e.g., a plate heat exchanger), and a liquid outlet of the carrier 1 is in communication with a liquid inlet of the second tank body 39. In this way, the liquid in the second tank body 39 is pumped out by the water pump 201, is then cooled by heat exchange after passing through the heat exchanger 43, and is then delivered to the carrier 1, so that cold compress is applied to a part of a human body wrapped by the carrier 1 via the carrier 1. The liquid in the carrier 1 also flows back to the second tank body 39 and is then pumped out by the pumping device 24 again to complete a cooling cycle. As the liquid participating in the cooling cycle is the liquid in the second tank body 39, the liquid is replenished to the second tank body 39 from the liquid in the first tank body 38 only when the liquid in the second tank body 39 is insufficient. For example, when the cold-hot compress device is turned on and in use, there is no liquid in the carrier 1 at this time, and the liquid in the second tank body 39 is pumped out into the carrier 1. The liquid in the second tank body 39 is insufficient at this time, so the liquid can be replenished to the second tank body 39 from the first tank body 38. When the liquid in the carrier 1 flows back to the second tank body 39, the entire cooling cycle is filled with the liquid, and the liquid does not need to be replenished to the second tank body 39 from the first tank body 38. Therefore, the liquid in the first tank body 38 does not participate in the cooling cycle, thereby reducing a total amount of the liquid participating in the cooling cycle without reducing a total volume of the storage tank 300. A smaller amount of the liquid can be cooled to a required temperature more quickly, thereby improving a cooling efficiency. When the liquid in the second tank body 39 is consumed, the liquid can be replenished to the second tank body 39 from the first tank body 38. In order to further improve the cooling efficiency, a volume of the second tank body 39 is set to be smaller than that of the first tank body 38.

In this embodiment, a height difference is formed between the first tank body 38 and the second tank body 39, so that the liquid in the first tank body 38 flows to the second tank body 39 under an action of gravity. In this way, a purpose of automatic liquid replenishment can be achieved. As a simple replacement of this embodiment, an electromagnetic valve can be disposed at a communication position between the first tank body 38 and the second tank body 39, and a liquid level sensor can be disposed in the second tank body 39. A liquid level height in the second tank body 39 is detected by the liquid level sensor, and when the liquid level height in the second tank body 39 is lower than a preset value, the electromagnetic valve can be opened to replenish the liquid to the second tank body 39.

Specifically, the first tank body 38 is in communication with the second tank body 39 via a pipeline 301. As there is a height difference between the first tank body 38 and the second tank body 39, the pipeline 301 is in communication with a bottom of the first tank body 38 and a side of the second tank body 39.

In addition, after the liquid in the second tank body 39 is consumed, an air cavity is formed in a section where the liquid is consumed, which will cause the liquid in the first tank body 38 to be unable to be replenished into the second tank body 39. Therefore, the second tank body 39 needs to be exhausted, so that initial air in the air cavity can be discharged out of the second tank body 39 when the liquid in the first tank body 38 enters into the air cavity. Therefore, the second tank body 39 comprises an exhaust hole 390. The exhaust hole 390 is in communication with the first tank body 38 via an exhaust pipe 302, and a communication position of the exhaust pipe 302 and the first tank body 38 is higher than a maximum liquid level line of the first tank body 38. In this way, it can be ensured that a side of the exhaust pipe 302 in communication with the first tank body 38 will not be blocked by the liquid in the first tank body 38, resulting in a failure of air exhaust function.

In addition, in this embodiment, a one-way valve 303 is disposed between the heat exchanger 43 and the liquid inlet of the carrier 1. In this way, one-way communication between the heat exchanger 43 and the carrier 1 can be achieved, and air is prevented from entering into the second tank body 39 through the heat exchanger 43 after the air pump 30 in embodiment 1 is added to the cold-hot compress device.

In this embodiment, a main body 130 of the carrier 1 comprises an outer layer 16, and the inflatable layer 11 and the working layer 12 for receiving the liquid are disposed on a side of the outer layer 16 facing skin of a human body.

As the working layer 12 of the cold-hot compress device plays a role in cold-hot compress and the inflatable layer 11 only assists the working layer 12 in enabling the working layer 12 to fit more closely with the skin of the human body to achieve a better effect, the inflatable layer 11 and the working layer 12 are stacked along a thickness direction of a cavity 13 of the main body 130, and the working layer 12 is disposed on an inner side of the inflatable layer 11. In this way, the working layer 12 can directly contact the skin of the human body, and cold-hot compress effects will not decrease due to an arrangement of the inflatable layer 11. The inflatable layer 11 is disposed on an outer side of the working layer 12, and when the inflatable layer 11 expands, the working layer 12 can approach in a direction close to the skin of the human body under extrusion from the inflatable layer 11, so as to achieve a better fitting effect.

In addition, during long-term use, a surface fabric of the working layer 12 is stretched and deformed due to repeated filling and discharging of the liquid, resulting in an increase in a volume of the working layer 12 and an increase in a used amount of the liquid. The increased used amount of the liquid will lead to a decrease in an efficiency of the cold-hot compress. In order to solve this problem, fabrics of the working layer 12 are divided into one or more cloth base layers 121 and one or more thermoplastic polyurethane (TPU) layers 122, and the one or more TPU layers 122 are disposed on one or more inner sides of the one or more cloth base layers 121. Moreover, the one or more TPU layers 122 in an upper fabric and a lower fabric of the working layer 12 are connected at a certain distance by pressing along an extending direction of the working layer 12 to form a connection section 125. That is, the one or more TPU layers 122 in the upper fabric and the lower fabric of the working layer 12 are connected to define the volume of the working layer 12. TPU material of the one or more TPU layers 122 has good tensile and deformation resistance. Therefore, during a liquid filling and discharging process of the working layer 12, even if the one or more cloth base layers 121 are stretched and deformed, the volume of the working layer 12 can be maintained at a relatively stable level due to a performance of the tensile and deformation resistance of the one or more TPU layers 122. Thus, a situation in which the volume of the working layer 12 increases and the used amount of the liquid increases will not occur after the long-term use.

In this embodiment, the main body 130 has a cavity 13 for integrally wrapping a leg, a waist, an arm, an upper body or a whole body, and a shape of the cavity 13 is designed for a shape according to different parts of a human body. Specifically, the arm of the human body has a curve that is wide at an upper part and narrow at a lower part so as to correspond to the carrier 1 for wrapping the arm of the human body. When the carrier 1 for wrapping the arm of the human body is unfolded into a flat state, a width of the carrier 1 has a structure that gradually decreases from a middle to a lower end, so that the cavity 13 corresponding to the shape of the arm can be formed when the carrier 1 is wrapped around the arm of the human body by winding. Moreover, a connection structure 140 is disposed on the main body 130 for changing the carrier 1 between an unfolded state and a wrapped state. Specifically, the connection structure 140 can adopt a quick disassembly and assembly design such as a zipper structure or a hook and loop fasterner structure. It should be noted that the carrier 1 for wrapping the leg can be divided into a carrier 1 for wrapping a single leg and a carrier 1 for wrapping two legs, wherein the carrier 1 for wrapping the two legs can also be divided into a carrier 1 for wrapping two legs simultaneously in a form of trousers or the carrier 1 for wrapping two legs respectively in two independent forms. It should be noted that the cavity 13 can also be directly disposed on the carrier 1, rather than being formed into the cavity 13 when the carrier 1 is wrapped around the human body. For example, the carrier 1 directly has two cavities 13 for wrapping the legs when being in the form of the trousers.

A specific structure of the working layer 12 will be described below. In order to achieve liquid circulation, a liquid inlet 14 and a liquid outlet 15 that are in communication with the working layer 12 are further disposed on the main body 130. The liquid flows into the working layer 12 from the liquid inlet 14 from the main unit 2 and then flows into the main unit 2 from the liquid outlet 15 to achieve the liquid circulation. In order to avoid a situation in which a flow path of the liquid between the liquid inlet 14 and the liquid outlet 15 is too short, resulting in the liquid failing to pass through the entire working layer 12, a flow channel 126 that the liquid flows through needs to be limited to enable the flow channel 126 that the liquid flows through to be increased as much as possible, and a contact time and a contact area between the liquid and a surface skin of the human body can be increased, thereby improving an effect of the cold-hot compress. For this purpose, a first indentation 127 is disposed in the working layer 12, and the first indentation 127 is also formed by connecting the TPU layers 122 in the upper fabric and the lower fabric of the working layer 12 by pressing, so that the flow channel 126 in communication with the liquid inlet 14 and the liquid outlet 15 is defined in the working layer 12 by the first indentation 127. The flow channel 126 is disposed in a winding arrangement. The phrase "winding arrangement" means that the flow channel 126 comprises at least one corner, and the liquid turns by a certain angle when flowing through the at least one corner. A number of the at least one corner can be set according to different parts of the body. For example, for the carrier 1 for wrapping the arm and the waist, the number of the at least one corner can be set to 1. For the carrier 1 for wrapping the upper body or the leg, the number of the at least one corner needs to be increased.

In addition, for some specific positions, for example, a width of the flow channel 126 of the carrier 1 for wrapping the upper body in a position of a chest of the upper body is relatively wide, and the liquid may be uneven locally when flowing through this part. That is, the liquid is more in some sections and less in some sections. Alternatively a flow rate of the liquid in one section may be too fast, resulting in a contact time between the liquid and the surface skin of the human body being too short. In this case, a second indentation 123 is further disposed in the working layer 12, and a certain distance is formed between the second indentation 123 and the first indentation 127 at a position where the second indentation 123 is located, so as to divide the liquid flowing through the second indentation 123 into two parts. In this way, the liquid can flow out from both sides of the second indentation 123, and the second indentation 123 can block the liquid at the same time, so that the liquid is more uniform when passing through the one section, and the contact time with the surface skin of the human body can be increased accordingly. A main purpose of arranging the first indentation 127 and the second indentation 123 is to allow the liquid to flow through all sections in the working layer 12 of the carrier 1. Whether the first indentation 127 is present depends on a width of the working layer 12. If the width of the working layer 12 is relatively wide and a flow amount of the liquid flowing into the working layer 12 is limited, the limited liquid cannot fill the entire working layer 12. At this time, the first indentation 127 and the second indentation 123 are needed to divide the working layer 12 into winding flow channels to reduce the width of the flow channel 126. Thus, the liquid can flow through the largest possible section. If the inherent width of the working layer 12 is relatively small, it is not necessary to have the first indentation 127 and the second indentation 123.

In addition, in order to reduce a used amount of the liquid, so as to achieve a rapid temperature rise and temperature fall of the carrier 1, press-fit points 124 for reducing a volume of the flow channel 126 are disposed in an array in the working layer 12. As the press-fit points 124 occupy a part of a space in the flow channel 126, the volume in the flow channel 126 is reduced, and the used amount of liquid can be reduced naturally. Intervals between the press-fit points 124 and sizes of the press-fit points 124 can be set according to different parts of the human body corresponding to the flow channel 126. The larger the intervals, the lower a flow resistance of the liquid caused by the press-fit points 124, and the lower the flow rate of the liquid flowing through a part of the different parts. Conversely, the smaller the intervals, the higher the flow resistance of the liquid caused by the press-fit points 124, and the higher the flow rate of the liquid flowing through the part of the different parts. Therefore, sizes of the intervals can be set corresponding to actual needs to obtain the best effect. It should be noted that the sizes of the intervals between the press-fit points 124 and the sizes of the press-fit points 124 can be set according to the different parts of the human body corresponding to the flow channel 126, which means that the sizes of the intervals between the press-fit points 124 can be different in different carriers 1 or the sizes of the intervals between the press-fit points 124 can also be different in different locations within the same carrier 1. For example, in the carrier 1 for wrapping two legs, the sizes of the intervals between the press-fit points 124 are different in different sections.

A structure of the inflatable layer 11 will be described below. The inflatable layer 11 is composed of a plurality of independent airbags 111, and each of the plurality of independent airbags 111 respectively comprises an air hole 112 for air intake and air exhaust. Both of the air intake and the air exhaust can be achieved through a same air hole 112, and the pumping device 24 switches working modes to achieve the air intake or the air exhaust through the air hole 112, so as to achieve air inflation and deflation processes of the plurality of independent airbags 111. The inflatable layer 11 is divided into the plurality of independent airbags 111 so as to accelerate the air inflation and deflation speed of each of the plurality of independent airbags 111, and at the same time, a certain airbag or certain several airbags 111 of the plurality of independent airbags 111 can be inflated and deflated independently according to different mode selections. Alternatively, the plurality of airbags 111 can be inflated and deflated according to a certain rule to achieve a massage effect. In addition, each of the plurality of independent airbags 111 has a section 113 combined with another of the plurality of independent airbags 111. Two adjacent ones of the plurality of independent airbags 111 are joined via the section 113 to form the complete inflatable layer 111.

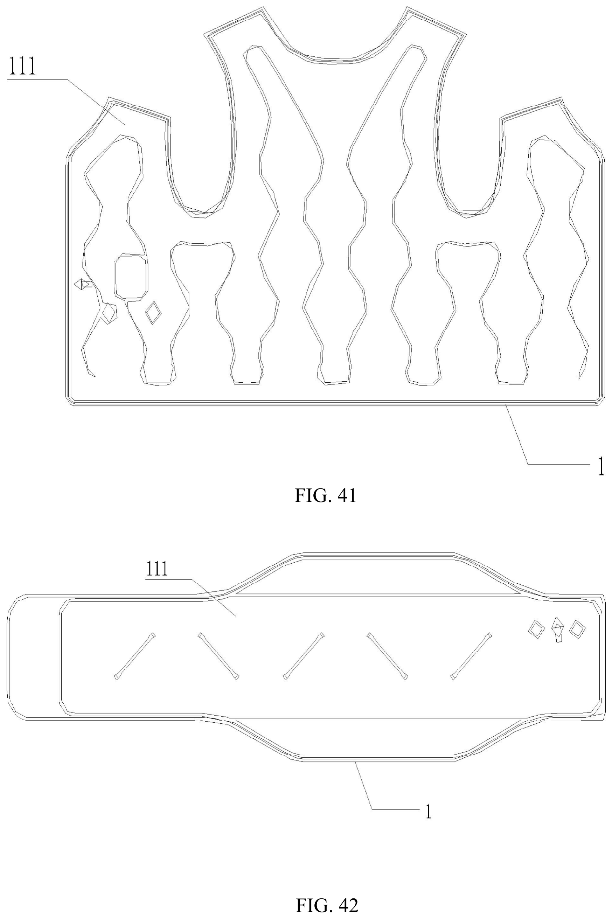

Not all of the inflatable layer 11 of the carrier 1 is composed of the plurality of independent airbags 111, and the inflatable layer 11 of the carrier 1 may instead be composed of only one of the plurality of independent airbags 111. For example, the carrier 1 for wrapping the upper body or the waist only has one of the plurality of independent airbags 111, as shown in FIGS. 41 and 42.

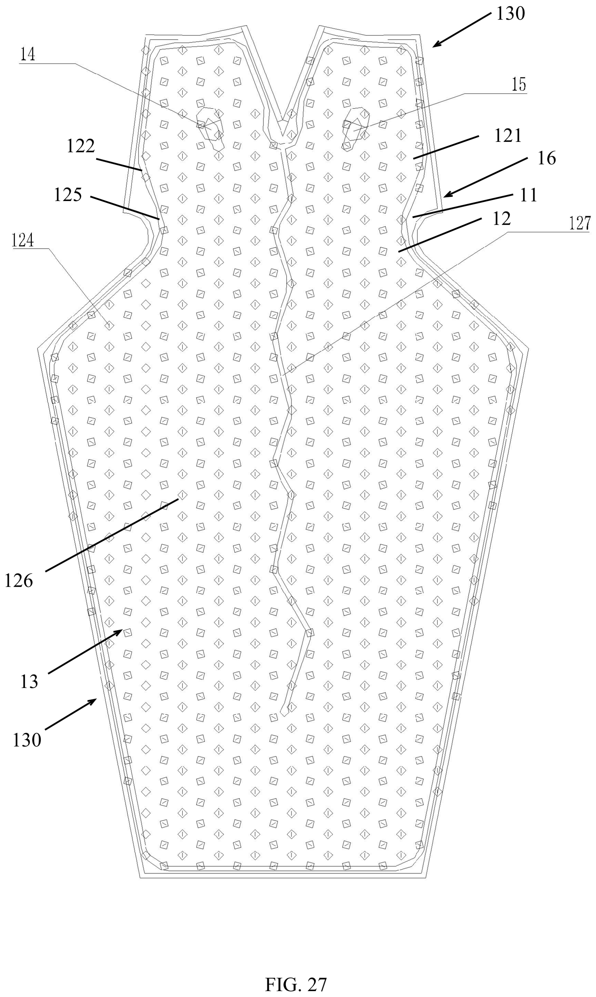

FIGS. 14-19 are only used to illustrate parts of the human body possible for being wrapped using the carrier 1, and the carrier 1 in FIGS. 14-19 is simplified, so it is different from the various specific styles of the carrier 1 in FIGS. 27-38.

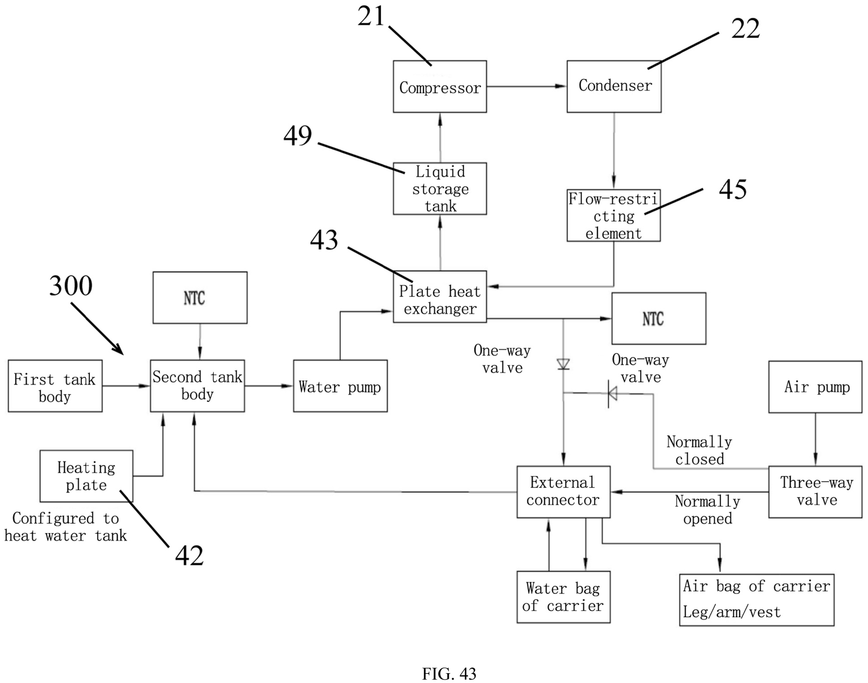

A principle diagram of the aforementioned cold-hot compress component is shown in FIGS. 43-46. When the cold-hot compress component works in a cooling mode, the water pump 201 pumps out the liquid in the second tank body 39, and the liquid is cooled by heat exchange through the heat exchanger 43 and then delivered to the working layer 12 of the carrier 1. The heat exchanger 43, the compressor 21, the condenser 22, and the flow-restricting element 45 form a cold-hot component, and heat dissipation is performed via a heat dissipation fan 26 of a radiator 25 to input a refrigerant into the heat exchanger 43 again after cooling. In addition, a liquid storage tank 49 is connected in series between the heat exchanger 43 and the compressor 21 to dynamically adjust an amount of the refrigerant in the cold-hot component, so as to prevent the refrigerant in a liquid state from entering the compressor 21 and thus prevent the compressor 21 from liquid absorption by storing a liquefied refrigerant via the liquid storage tank 49. The liquid storage tank 49 can store part of the refrigerant in the liquid storage tank 49 when a demand for the refrigerant is small and can release the stored refrigerant when the demand for the refrigerant is large, so as to achieve dynamic adjustment. In the cooling mode, the normally open line of the three-way valve 40 is opened, and the air is delivered to the inflatable layer 11 of the carrier 1.

When the cold-hot compress component works in a heating mode, the liquid in the second tank body 39 is heated via a heating element 42 (e.g., a heating plate), and a temperature of the liquid in the second tank body 39 is detected via a temperature sensor (e.g., negative temperature coefficient (NTC) sensor), so as to control a heating temperature of the liquid. The heated liquid is also pumped out by the water pump 201 and then delivered to the working layer 12 of the carrier 1. In the heating mode, the normally open line of the three-way valve 40 is opened, and the air is delivered to the inflatable layer 11 of the carrier 1.

When being shut down for water drainage, the normally closed line of the three-way valve 40 is opened, and the air is delivered to the working layer 12 of the carrier 1.

### Embodiment 10

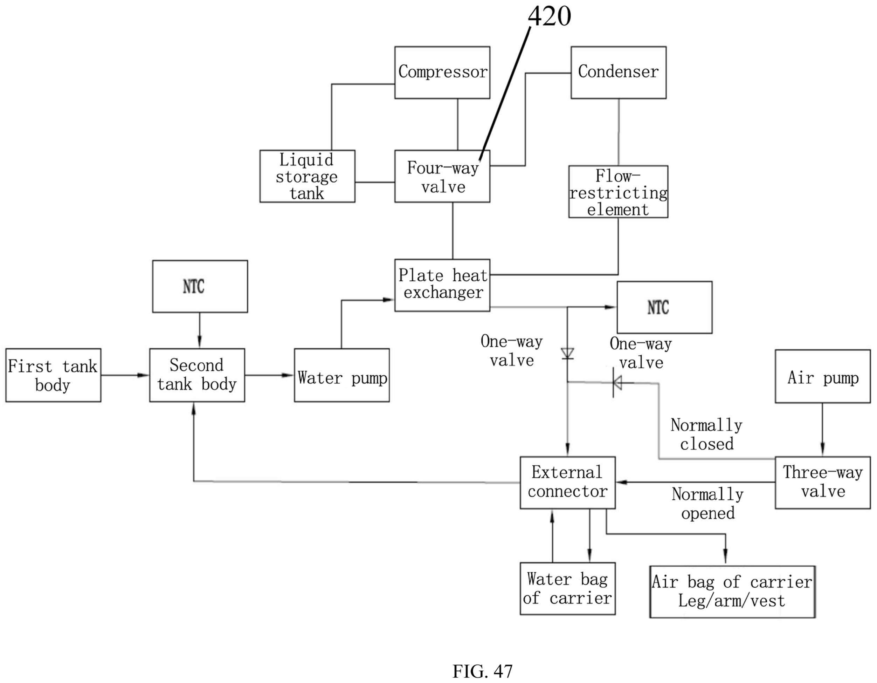

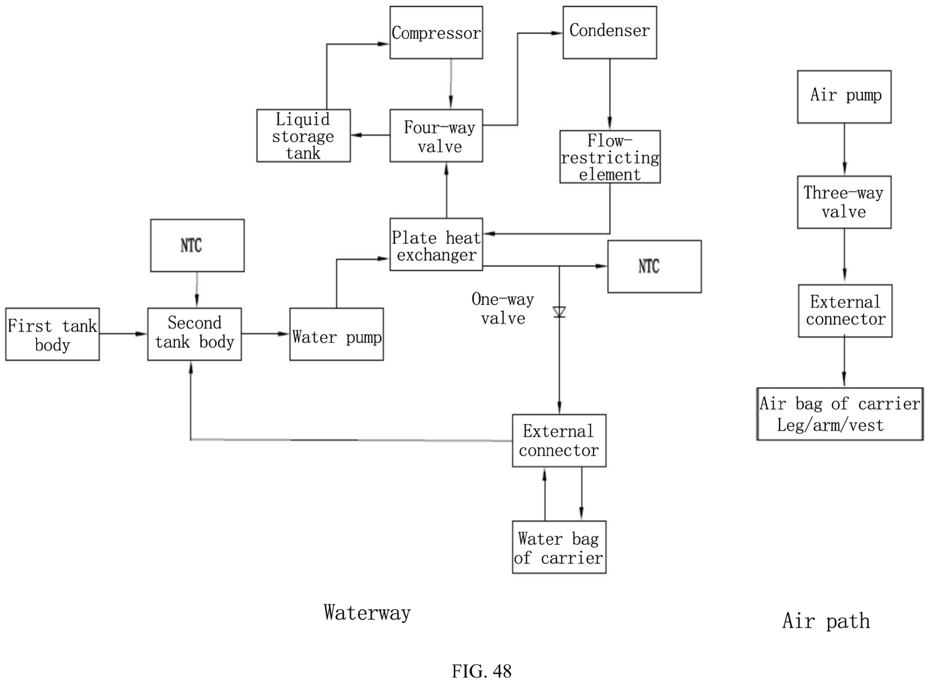

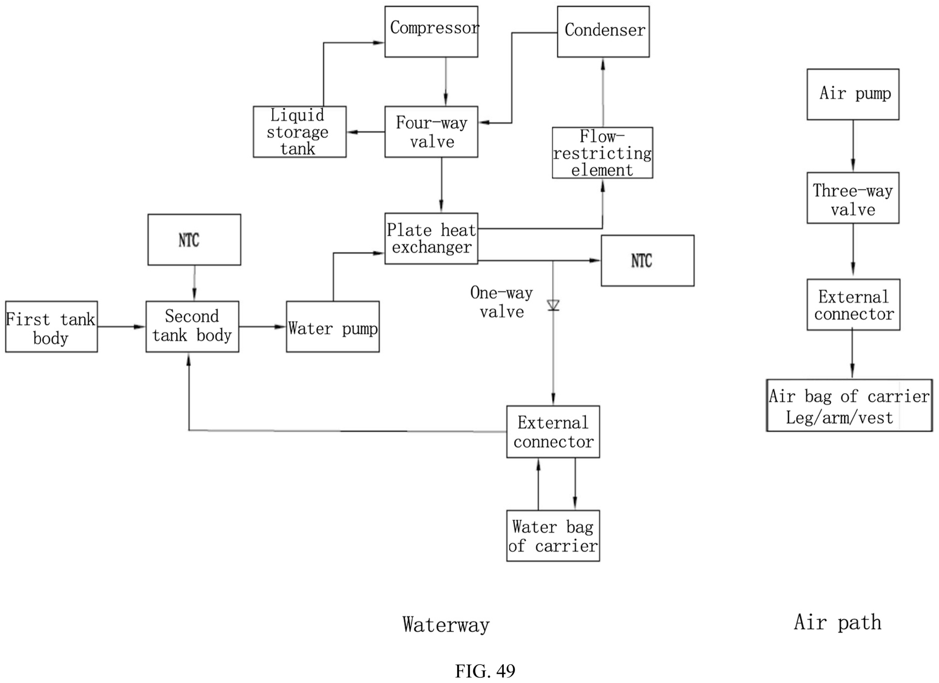

Referring to FIGS. 47-49, this embodiment differs from Embodiment 9 in that, in Embodiment 9, the heat exchanger 43, the liquid storage tank 49, the compressor 21, the condenser 22 and the flow-restricting element 45 are connected in sequence. In this embodiment, a four-way valve 420 is added, the heat exchanger 43 is connected to a first end of the four-way valve, the liquid storage tank 49 and the compressor 21 are connected in series between a second end and a third end of the four-way valve 420, and the condenser 22 and the flow-restricting element 45 are connected to a fourth end of the four-way valve 420. With this arrangement, the compressor 21 can also participate in heating in the heating mode. That is, after the water pump 201 pumps out the heated liquid in the second tank body 39, the compressor 21 heats the flowing liquid via the heat exchanger 43 when the liquid flows through the heat exchanger 43.

In conclusion, in the cold-hot compress device of the present disclosure, through an arrangement of the circulation component, the medium is pumped by the pumping device 24 from the storage tank 300, exchanges heat with the cold-hot component, and then is input into the carrier 1 to exchange heat with the nursing part, and finally returns to the storage tank 300. The temperature of the medium in the delivery pipe 400 is adjusted through the cooling and heating of the cold-hot component, so that the user can realize the cold-hot compress function after assembling the carrier 1. Compared with the traditional semiconductor cooling and heating, the cold-hot component composed of the compressor 21, the evaporator, the expansion valve 451 and the condenser 22 has a wider application range and a larger load, and can be applied to large-area and high-power cold-hot compress operations.

The present invention may be summarized as follows: The present disclosure provides a drainage method for a cold-hot compress device, the cold-hot compress device comprises a carrier and a main unit; the carrier comprises an inflatable layer and a working layer, a pumping device is disposed in the main unit, and the pumping device is connected to the inflatable layer and the working layer via a delivery pipe to deliver air to the inflatable layer and deliver liquid or the air to the working layer; and the drainage method comprises a step of delivering the air to the working layer via the pumping device to discharge the liquid in the working layer. The present disclosure further provides a drainage structure adopting the aforementioned method. The present disclosure further provides a cold-hot compress device, it comprises the drainage structure, it further comprises a storage tank and a heat exchanger, the pumping device is used to deliver the liquid in the storage tank to the working layer via the waterway delivery pipe, and the waterway delivery pipe exchanges heat with the heat exchanger.

The present invention may also be summarized as follows:
1. A drainage method for a cold-hot compress device, characterized in that: the cold-hot compress device comprises a carrier and a main unit; the carrier comprises an inflatable layer and a working layer, a pumping device is disposed in the main unit, and the pumping device is connected to the inflatable layer and the working layer via a delivery pipe to deliver air to the inflatable layer and deliver liquid or the air to the working layer; and
   the drainage method comprises a step of delivering the air to the working layer via the pumping device to discharge the liquid in the working layer.
2. A drainage structure for a cold-hot compress device, characterized in that: the cold-hot compress device comprises a carrier and a main unit, the carrier comprises an inflatable layer and a working layer, a pumping device is disposed in the main unit, the pumping device is respectively connected to the inflatable layer and the working layer via an air path delivery pipe and a waterway delivery pipe; and
   the pumping device is further connected to the waterway delivery pipe via a control valve, so as to deliver air to the working layer via the waterway delivery pipe when the control valve is opened.
3. The drainage structure for the cold-hot compress device according to item 2, characterized in that: the control valve is a three-way valve with one inlet and two outlets, the inlet of the three-way valve is in communication with an air outlet of the pumping device, a first outlet is connected to the air path delivery pipe, and a second outlet is connected to the waterway delivery pipe.
4. The drainage structure for the cold-hot compress device according to claim 2 and/or 3, characterized in that: the pumping device comprises an air pump and a water pump, and a water outlet of the water pump is connected to the waterway delivery pipe; or the pumping device is an air-liquid dual-purpose pump.
5. A cold-hot compress device, characterized in that: it comprises the drainage structure according to any one or more of items 2-4, it further comprises a storage tank and a heat exchanger, the pumping device is used to deliver the liquid in the storage tank to the working layer via the waterway delivery pipe, and the waterway delivery pipe exchanges heat with the heat exchanger.
6. The cold-hot compress device according to item 5, characterized in that: the storage tank comprises a first tank body and a second tank body, and the first tank body is in communication with the second tank body; and the first tank body is used for replenishing the liquid to the second tank body, and the second tank body is in communication with the working layer via the pumping device to output the liquid to the working layer or receive the liquid from the working layer.
7. The cold-hot compress device according to item 6, characterized in that: the second tank body comprises an exhaust hole.
8. The cold-hot compress device according to item 7, characterized in that: the exhaust hole is in communication with the first tank body via an exhaust pipe, and a communication position between the exhaust pipe and the first tank body is higher than a maximum liquid level line of the first tank body.
9. The cold-hot compress device according to any one or more of items 6 to 8, characterized in that: a height difference is formed between the first tank body and the second tank body, so that the liquid in the first tank body flows to the second tank body under an action of gravity.
10. The cold-hot compress device according to any one or more of items 6 to 9, characterized in that: a liquid inlet of the pumping device is in communication with a liquid outlet of the second tank body, a liquid outlet of the pumping device is in communication with a liquid inlet of the carrier via the heat exchanger, and a liquid outlet of the carrier is in communication with a liquid inlet of the second tank body.
11. The cold-hot compress device according to item 10, characterized in that: a one-way valve is disposed between the heat exchanger and the liquid inlet of the carrier.
12. The cold-hot compress device according to any one or more of items 6 to 11, characterized in that: a volume of the first tank body is larger than that of the second tank body.
13. The cold-hot compress device according to any one or more of items 5-12, characterized in that: the carrier comprises a main body configured for integrally wrapping a leg, a waist, an arm, an upper body or a whole body.
14. The cold-hot compress device according to item 13, characterized in that: the main body comprises an outer layer, and the inflatable layer and the working layer are disposed on a side of the outer layer facing skin of a human body.
15. The cold-hot compress device according to any one or more of items 5 to 14, characterized in that: fabric of the working layer is divided into cloth base layers and thermoplastic polyurethane (TPU) layers, and the TPU layers in an upper fabric and a lower fabric of the working layer are connected by pressing at a preset distance along an extending direction of the working layer.
16. The cold-hot compress device according to any one or more of items 13 to 15, characterized in that: the main body has a cavity for integrally wrapping the leg, the waist, the arm, the upper body or the whole body, and a shape of the cavity is set according to a shape of different parts of a human body.
17. The cold-hot compress device according to any one or more of items 13 to 16, characterized in that: the main body further comprises a liquid inlet and a liquid outlet that are in communication with the working layer.
18. The cold-hot compress device according to any one or more of items 13 to 17, characterized in that: a first indentation is disposed in the working layer, and the first indentation is used for defining a flow channel in communication with the liquid inlet of the main body to the liquid outlet of the main body in the working layer; and the flow channel is disposed in a winding manner.
19. The cold-hot compress device according to item 18, characterized in that: a second indentation is further disposed in the working layer, and a preset distance is formed between the second indentation and the first indentation at a position where the second indentation is located, so as to divide the liquid flowing through the second indentation into two parts.
20. The cold-hot compress device according to item 19, characterized in that: the first indentation and the second indentation are curved press-fit lines.
21. The cold-hot compress device according to any one or more of items 18 to 20, characterized in that: press-fit points for reducing a volume of the flow channel are disposed in an array in the working layer.
22. The cold-hot compress device according to item 21, characterized in that: sizes of intervals between the press-fit points are set according to different parts of a human body corresponding to the flow channel, the larger the intervals, the lower a flow resistance of the liquid flowing through a part of the different parts, and conversely, the smaller the intervals, the higher the flow resistance of the liquid flowing through the part of the different parts.
23. The cold-hot compress device according to any one or more of items 5 to 22, characterized in that: the inflatable layer is composed of a plurality of independent airbags, and each of the plurality of independent airbags respectively comprises an air hole for air intake and air exhaust.

The preceding description is merely a specific embodiment of the present disclosure, but the design and the concept of the present disclosure is not limited thereto. It is intended that the protective scope of the present disclosure cover any non-substantial modification provided it is made using the concept.

## Claims

1. A drainage method for a cold-hot compress device, **characterized in that**: the cold-hot compress device comprises a carrier and a main unit; the carrier comprises an inflatable layer and a working layer, a pumping device is disposed in the main unit, and the pumping device is connected to the inflatable layer and the working layer via a delivery pipe to deliver air to the inflatable layer and deliver liquid or the air to the working layer; and
the drainage method comprises a step of delivering the air to the working layer via the pumping device to discharge the liquid in the working layer.

2. A drainage structure for a cold-hot compress device, **characterized in that**: the cold-hot compress device comprises a carrier and a main unit, the carrier comprises an inflatable layer and a working layer, a pumping device is disposed in the main unit, the pumping device is respectively connected to the inflatable layer and the working layer via an air path delivery pipe and a waterway delivery pipe; and
the pumping device is further connected to the waterway delivery pipe via a control valve, so as to deliver air to the working layer via the waterway delivery pipe when the control valve is opened.

3. The drainage structure for the cold-hot compress device according to claim 2, **characterized in that**: the control valve is a three-way valve with one inlet and two outlets, the inlet of the three-way valve is in communication with an air outlet of the pumping device, a first outlet is connected to the air path delivery pipe, and a second outlet is connected to the waterway delivery pipe.

4. The drainage structure for the cold-hot compress device according to claim 2 and/or 3, **characterized in that**: the pumping device comprises an air pump and a water pump, and a water outlet of the water pump is connected to the waterway delivery pipe; or the pumping device is an air-liquid dual-purpose pump.

5. A cold-hot compress device, **characterized in that**: it comprises the drainage structure according to any one or more of claims 2-4, it further comprises a storage tank and a heat exchanger, the pumping device is used to deliver the liquid in the storage tank to the working layer via the waterway delivery pipe, and the waterway delivery pipe exchanges heat with the heat exchanger.

6. The cold-hot compress device according to claim 5, **characterized in that**: the storage tank comprises a first tank body and a second tank body, and the first tank body is in communication with the second tank body; and the first tank body is used for replenishing the liquid to the second tank body, and the second tank body is in communication with the working layer via the pumping device to output the liquid to the working layer or receive the liquid from the working layer; wherein preferably the second tank body comprises an exhaust hole; wherein preferably the exhaust hole is in communication with the first tank body via an exhaust pipe, and a communication position between the exhaust pipe and the first tank body is higher than a maximum liquid level line of the first tank body; wherein preferably a height difference is formed between the first tank body and the second tank body, so that the liquid in the first tank body flows to the second tank body under an action of gravity.

7. The cold-hot compress device according to claim 6, **characterized in that**: a liquid inlet of the pumping device is in communication with a liquid outlet of the second tank body, a liquid outlet of the pumping device is in communication with a liquid inlet of the carrier via the heat exchanger, and a liquid outlet of the carrier is in communication with a liquid inlet of the second tank body; wherein preferably a one-way valve is disposed between the heat exchanger and the liquid inlet of the carrier.

8. The cold-hot compress device according to any one or more of claims 6 to 7, **characterized in that**: a volume of the first tank body is larger than that of the second tank body.

9. The cold-hot compress device according to any one or more of claims 5 to 8, **characterized in that**: the carrier comprises a main body configured for integrally wrapping a leg, a waist, an arm, an upper body or a whole body; wherein preferably the main body comprises an outer layer, and the inflatable layer and the working layer are disposed on a side of the outer layer facing skin of a human body.

10. The cold-hot compress device according to any one or more of claims 5 to 9, **characterized in that**: fabric of the working layer is divided into cloth base layers and thermoplastic polyurethane (TPU) layers, and the TPU layers in an upper fabric and a lower fabric of the working layer are connected by pressing at a preset distance along an extending direction of the working layer.

11. The cold-hot compress device according to any one or more of claims 9 to 10, **characterized in that**: the main body has a cavity for integrally wrapping the leg, the waist, the arm, the upper body or the whole body, and a shape of the cavity is set according to a shape of different parts of a human body.

12. The cold-hot compress device according to any one or more of claims 9 to 11, **characterized in that**: the main body further comprises a liquid inlet and a liquid outlet that are in communication with the working layer.

13. The cold-hot compress device according to any one or more of claims 9 to 12, **characterized in that**: a first indentation is disposed in the working layer, and the first indentation is used for defining a flow channel in communication with the liquid inlet of the main body to the liquid outlet of the main body in the working layer; and the flow channel is disposed in a winding manner; wherein preferably a second indentation is further disposed in the working layer, and a preset distance is formed between the second indentation and the first indentation at a position where the second indentation is located, so as to divide the liquid flowing through the second indentation into two parts; wherein more preferably the first indentation and the second indentation are curved press-fit lines.

14. The cold-hot compress device according to claim 13, **characterized in that**: press-fit points for reducing a volume of the flow channel are disposed in an array in the working layer; wherein preferably sizes of intervals between the press-fit points are set according to different parts of a human body corresponding to the flow channel, the larger the intervals, the lower a flow resistance of the liquid flowing through a part of the different parts, and conversely, the smaller the intervals, the higher the flow resistance of the liquid flowing through the part of the different parts.

15. The cold-hot compress device according to any one or more of claims 5 to 14, **characterized in that**: the inflatable layer is composed of a plurality of independent airbags, and each of the plurality of independent airbags respectively comprises an air hole for air intake and air exhaust.
